# EUROPEAN PATENT APPLICATION

(11) **EP 4 122 922 A1**
(43) Date of publication of application: **25.01.2023**
(21) Application number: 21770969.0
(22) Date of filing: 16.03.2021
(51) Int. Cl.: C07D 239/70, C07D 487/08, C07D 401/14, A61K 31/517, A61P 35/00

(54) **FUSED BICYCLIC DERIVATIVE, PREPARATION METHOD THEREFOR, AND PHARMACEUTICAL USE THEREOF**

(30) Priority: 17.03.2020 CN 202010185224; 18.05.2020 CN 202010418453; 04.03.2021 CN 202110241159
(71) Applicant: Jiangsu Hengrui Pharmaceuticals Co., Ltd., Lianyungang, Jiangsu 222047 (CN); Shanghai Hengrui Pharmaceutical Co., Ltd., Shanghai 200245 (CN)
(72) Inventor: LI, Xin, Shanghai 200245 (CN); ZHANG, Zhigao, Shanghai 200245 (CN); CHEN, Yang, Shanghai 200245 (CN); LI, Zhihao, Shanghai 200245 (CN); HE, Feng, Shanghai 200245 (CN); TAO, Weikang, Shanghai 200245 (CN)
(74) Representative: Regimbeau
(86) International application number: PCT/CN2021/081033
(87) International publication number: WO 2021/185238

(57) **Abstract**

A fused bicyclic derivative shown in general formula (I), a preparation method therefor, a pharmaceutical composition comprising the derivative, and a use thereof as a therapeutic agent, particularly the use thereof as an AKT1/2/3 (AKT pan) inhibitor and the use in the preparation of a medication for treating and/or preventing tumors. Each group in general formula (I) is as defined in the description.

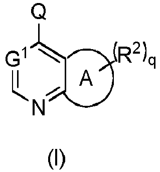

## Description

### TECHNICAL FIELD

The present disclosure belongs to the field of pharmaceutics, and relates to a fused bicyclic derivative of general formula (I), a method for preparing the derivative, a pharmaceutical composition comprising the derivative, and use of the derivative as a therapeutic agent, specifically use as an AKT 1/2/3 (AKT pan) inhibitor and use in preparing a medicament for treating and preventing a tumor.

### BACKGROUND

Protein kinase B (PKB, also known as AKT) is central to PI3K/AKT/mTOR signaling in cells, and its function has important roles in cell growth, survival, differentiation and metabolism. The PI3K signaling pathway is involved in and regulates the expression of multiple oncogenes and anticancer genes, and over-activation of the PI3K/AKT signaling pathway has been proved to be associated with the development of multiple cancers.

In cells, AKT can be activated by a series of signals, including growth factors. When the receptor tyrosine kinase on the cell membrane is activated by a growth factor, downstream PI3K is activated, so that phosphatidylinositol-4,5-biphosphate (PIP2) is phosphorylated to form phosphatidylinositol-3,4,5-triphosphate (PIP3). Finally, phosphatidylinositol-dependent kinase 1 (PDK1) and AKT are recruited to the cell membrane, and then AKT is activated by PDK1. Variation in PI3K and deletion and variation in PTEN will continuously activate AKT protein, which results in continuous activation of this pathway. The main function of AKT in cells is to promote cell proliferation, cause the cells to be transformed from benign ones to malignant ones and promote cell movement and invasion, thereby causing the metastasis and dissemination of tumor cells; besides, the high-activity phosphorylated AKT can also inhibit apoptosis and participate in chemotherapy resistance mechanism and thereby influence the effect of clinical treatment. In clinical statistics, the proportion of tumors with high-activity AKT in each different tumor can reach 40% or more.

There are 3 subtypes of AKT enzyme (AKT1, AKT2 and AKT3), each of which has been shown to function differently *in vivo* in various studies. Signal pathways activated by AKT1 mainly regulate cell proliferation and survival, and AKT2 has such functions as participating in cell invasion and migration and insulin-regulated blood sugar metabolism pathway. Although the gene knockout mouse of AKT3 only shows functions related to embryonic brain development, the clinical research shows that the expression level of AKT3 is increased significantly in various tumors, such as breast cancer. In addition, *in vitro* studies before clinic use show that the breast cancer cells can generate drug resistance in the long-term treatment with an AKT1/2 selective inhibitor MK2206, and the expression level of AKT3 is increased remarkably in the drug resistant cells. Inhibitors targeting AKT have been studied clinically for many years. The selective inhibitors of AKT1/2, MK2206 (Merck) and BAY1125976 (Bayer), have not been clinically successful for reasons related to therapeutic effects, toxicity and the like. However, in recent years, AKT1/2/3(AKT pan) inhibitors AZD5363 (AZ) and GDC0068 (Roche) have made breakthroughs in clinical phase 2 trial, and their combination with other anticancer drugs has exhibited significant efficacy in the treatment of triple negative breast cancer, ER+ breast cancer and prostate cancer. At the present, the two AKT1/2/3(AKT pan) inhibitors AZD5363 and GDC0068 have successfully entered the phase 3 clinical trial.

The cancer statistics in 2018 shows that there are 18 million new cancer cases and 9.6 million cancer death cases in the world, and the annual cancer incidence rate is increasing. The top three cancers are lung cancer (11.6%), female breast cancer (11.6%) and prostate cancer (7.1%). In China, because of the large population, the number of new cases and that of death cases of female breast cancer account for 11.2% and 9.2%, respectively, of those worldwide, ranking among the top in the world; prostate cancer is a high-incidence cancer in the United States, and prostate cancer patients are expected to reach 11 million worldwide in 2022, about 3 million (28%) of which are from the United States. Disclosed patent applications of AKT inhibitors include WO2006/071819, US8377937, WO2008/075109, US2010120801 and WO2009006040.

### SUMMARY

The present disclosure is intended to provide a compound of general formula (I) or a tautomer, mesomer, racemate, enantiomer, diastereomer thereof or a mixture thereof, or a pharmaceutically acceptable salt thereof: 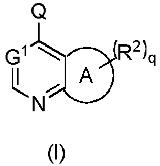 wherein:
Q is a group of formula (Qa) or (Qb): 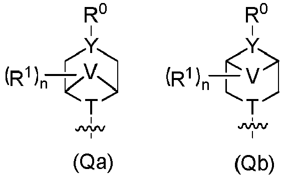
V is selected from the group consisting of -CH₂-, -C(CH₃)₂-, -CH₂CH₂-, -CH₂CH₂CH₂-, -CH₂OCH₂-, -CH₂SCH₂-, -CH₂S(O)CH₂-, -CH₂S(O)₂CH₂- and -CH₂N(R^{a})CH₂-;
Y is an N atom or CR³;
T is CH or an N atom; provided that when Y is CR³, T is an N atom;
R⁰ is -C(O)CHR⁵R⁶ or -C(O)NHCHR⁵R⁶;
R¹ are identical or different and are each independently selected from the group consisting of a hydrogen atom, halogen, alkyl, hydroxyalkyl, alkoxy, haloalkoxy and haloalkyl; ring A is 5-membered heterocyclyl, 5-membered cycloalkyl or 5-membered heteroaryl; G¹ is selected from the group consisting of CR⁴ and an N atom;
R² are identical or different and are each independently selected from the group consisting of a hydrogen atom, oxo, halogen, alkyl, alkenyl, alkynyl, alkoxy, haloalkyl, haloalkoxy, cyano, amino, nitro, hydroxy, hydroxyalkyl, cycloalkyl, heterocyclyl, aryl and heteroaryl;
R³ is selected from the group consisting of a hydrogen atom, halogen, alkyl, alkenyl, alkynyl, alkoxy, haloalkyl, haloalkoxy, cyano, amino, -NR⁷R⁸, nitro, hydroxy, hydroxyalkyl, cycloalkyl, heterocyclyl, aryl and heteroaryl, wherein the alkyl is optionally substituted with one or more substituents selected from the group consisting of amino, -NR⁷R⁸, halogen, alkoxy, haloalkyl, cyano, nitro, hydroxy, hydroxyalkyl, cycloalkyl, heterocyclyl, aryl and heteroaryl;
R^{a} is selected from the group consisting of a hydrogen atom, alkyl, haloalkyl, hydroxyalkyl, cycloalkyl, heterocyclyl, aryl and heteroaryl;
R⁴ is selected from the group consisting of a hydrogen atom, halogen, alkyl, alkenyl, alkynyl, alkoxy, haloalkyl, haloalkoxy, cyano, amino, nitro, hydroxy, hydroxyalkyl, cycloalkyl, heterocyclyl, aryl and heteroaryl;
R⁵ is selected from the group consisting of halogen, alkyl, alkenyl, alkynyl, alkoxy, haloalkyl, haloalkoxy, cyano, amino, nitro, hydroxy, hydroxyalkyl, cycloalkyl, heterocyclyl, aryl and heteroaryl, wherein the alkyl, alkenyl, alkynyl, alkoxy, hydroxyalkyl, cycloalkyl, heterocyclyl, aryl and heteroaryl are each independently optionally substituted with one or more substituents selected from the group consisting of -NR⁹R¹⁰, oxo, halogen, alkyl, alkenyl, alkynyl, alkoxy, haloalkyl, cyano, amino, nitro, hydroxy, hydroxyalkyl, cycloalkyl, heterocyclyl, aryl and heteroaryl;
R⁶ is selected from the group consisting of halogen, alkyl, alkenyl, alkynyl, alkoxy, haloalkyl, haloalkoxy, cyano, amino, nitro, hydroxy, hydroxyalkyl, cycloalkyl, heterocyclyl, aryl and heteroaryl, wherein the alkyl, alkenyl, alkynyl, alkoxy, hydroxyalkyl, cycloalkyl, heterocyclyl, aryl and heteroaryl are each independently optionally substituted with one or more substituents selected from the group consisting of oxo, halogen, alkyl, alkenyl, alkynyl, alkoxy, haloalkyl, cyano, amino, nitro, hydroxy, hydroxyalkyl, cycloalkyl, heterocyclyl, aryl and heteroaryl;
R⁷ and R⁸ are identical or different and are each independently selected from the group consisting of a hydrogen atom, halogen, alkyl, alkenyl, alkynyl, alkoxy, haloalkyl, haloalkoxy, cyano, amino, nitro, hydroxy, hydroxyalkyl, cycloalkyl, heterocyclyl, aryl and heteroaryl;
R⁹ and R¹⁰ are identical or different and are each independently selected from the group consisting of a hydrogen atom, halogen, alkyl, alkenyl, alkynyl, alkoxy, haloalkyl, haloalkoxy, cyano, amino, nitro, hydroxy, hydroxyalkyl, cycloalkyl, cycloalkylalkyl, heterocyclyl, aryl and heteroaryl;
n is 0, 1, 2, 3 or 4; and
q is 0, 1, 2, 3, 4 or 5.

In some preferred embodiments of the present disclosure, provided is the compound of general formula (I) or the tautomer, mesomer, racemate, enantiomer or diastereomer thereof or the mixture thereof, or the pharmaceutically acceptable salt thereof, which is a compound of general formula (IIaa), (IIbb) or (IIcc) or a tautomer, mesomer, racemate, enantiomer or diastereomer thereof or a mixture thereof, or a pharmaceutically acceptable salt thereof: 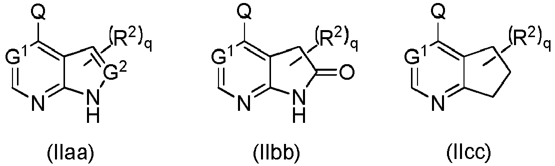 wherein:
G² is CR⁴ or an N atom; and
Q, G¹, R², R⁴ and q are defined as in general formula (I).

In some preferred embodiments of the present disclosure, provided is the compound of general formula (I) or (IIaa) or the tautomer, mesomer, racemate, enantiomer or diastereomer thereof or the mixture thereof, or the pharmaceutically acceptable salt thereof, which is a compound of general formula (IIaa-1) or (IIaa-2) or a tautomer, mesomer, racemate, enantiomer or diastereomer thereof or a mixture thereof, or a pharmaceutically acceptable salt thereof: 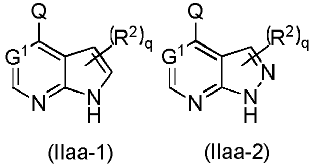 wherein:
Q, G¹, R² and q are defined as in general formula (I).

In some preferred embodiments of the present disclosure, provided is the compound of general formula (I) or (IIcc) or the tautomer, mesomer, racemate, enantiomer or diastereomer thereof or the mixture thereof, or the pharmaceutically acceptable salt thereof, which is a compound of general formula (IIcc-1) or a tautomer, mesomer, racemate, enantiomer or diastereomer thereof or a mixture thereof, or a pharmaceutically acceptable salt thereof: 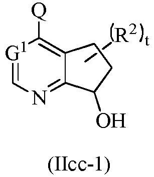 wherein:
t is 0, 1, 2, 3 or 4; and
Q, G¹ and R² are defined as in general formula (I).

In some preferred embodiments of the present disclosure, provided are the compounds of general formulas (I), (IIaa), (IIaa-1), (IIaa-2), (IIbb), (IIcc) and (IIcc-1) or the tautomers, mesomers, racemates, enantiomers or diastereomers thereof or the mixtures thereof, or the pharmaceutically acceptable salts thereof, wherein Q is the group of formula (Qb):

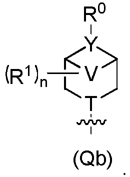

In some preferred embodiments of the present disclosure, provided are the compounds of general formulas (I), (IIaa), (IIaa-1), (IIaa-2), (IIbb), (IIcc) and (IIcc-1) or the tautomers, mesomers, racemates, enantiomers or diastereomers thereof or the mixtures thereof, or the pharmaceutically acceptable salts thereof, wherein V is selected from the group consisting of -CH₂-, -C(CH₃)₂-, -CH₂CH₂-, -CH₂CH₂CH₂- and -CH₂OCH₂-.

In some preferred embodiments of the present disclosure, provided are the compounds of general formulas (I), (IIaa), (IIaa-1), (IIaa-2), (IIbb), (IIcc) and (IIcc-1) or the tautomers, mesomers, racemates, enantiomers or diastereomers thereof or the mixtures thereof, or the pharmaceutically acceptable salts thereof, wherein V is selected from the group consisting of -CH₂-, -C(CH₃)₂-, -CH₂CH₂- and -CH₂CH₂CH₂-.

In some preferred embodiments of the present disclosure, provided are the compounds of general formulas (I), (IIaa), (IIaa-1), (IIaa-2), (IIbb), (IIcc) and (IIcc-1) or the tautomers, mesomers, racemates, enantiomers or diastereomers thereof or the mixtures thereof, or the pharmaceutically acceptable salts thereof, wherein T is an N atom.

In some preferred embodiments of the present disclosure, provided are the compounds of general formulas (I), (IIaa), (IIaa-1), (IIaa-2), (IIbb), (IIcc) and (IIcc-1) or the tautomers, mesomers, racemates, enantiomers or diastereomers thereof or the mixtures thereof, or the pharmaceutically acceptable salts thereof, wherein Y is an N atom, T is an N atom, and V is selected from the group consisting of -CH₂CH₂-, -CH₂CH₂CH₂- and -CH₂OCH₂-. In some preferred embodiments of the present disclosure, provided are the compounds of general formulas (I), (IIaa), (IIaa-1), (IIaa-2), (IIbb), (IIcc) and (IIcc-1) or the tautomers, mesomers, racemates, enantiomers or diastereomers thereof or the mixtures thereof, or the pharmaceutically acceptable salts thereof, wherein Y is an N atom, T is an N atom, and V is -CH₂CH₂-.

In some preferred embodiments of the present disclosure, provided are the compounds of general formulas (I), (IIaa), (IIaa-1), (IIaa-2), (IIbb), (IIcc) and (IIcc-1) or the tautomers, mesomers, racemates, enantiomers or diastereomers thereof or the mixtures thereof, or the pharmaceutically acceptable salts thereof, wherein G¹ is CH or an N atom.

In some preferred embodiments of the present disclosure, provided are the compounds of general formulas (I), (IIaa), (IIaa-1), (IIaa-2), (IIbb), (IIcc) and (IIcc-1) or the tautomers, mesomers, racemates, enantiomers or diastereomers thereof or the mixtures thereof, or the pharmaceutically acceptable salts thereof, wherein G¹ is an N atom.

In some preferred embodiments of the present disclosure, provided are the compounds of general formulas (I) and (IIaa) or the tautomers, mesomers, racemates, enantiomers or diastereomers thereof or the mixtures thereof, or the pharmaceutically acceptable salts thereof, wherein G² is CR⁴, and R⁴ is defined as in general formula (I).

In some embodiments of the present disclosure, provided are the compounds of general formulas (I), (IIaa), (IIaa-1), (IIaa-2), (IIbb), (IIcc) and (IIcc-1) or the tautomers, mesomers, racemates, enantiomers or diastereomers thereof or the mixtures thereof, or the pharmaceutically acceptable salts thereof, wherein R⁴ is a hydrogen atom.

In some preferred embodiments of the present disclosure, provided is the compound of general formula (I) or the tautomer, mesomer, racemate, enantiomer or diastereomer thereof or the mixture thereof, or the pharmaceutically acceptable salt thereof, which is a compound of general formula (IIIaa), (IIIbb) or (IIIcc) or a tautomer, mesomer, racemate, enantiomer or diastereomer thereof or a mixture thereof, or a pharmaceutically acceptable salt thereof: 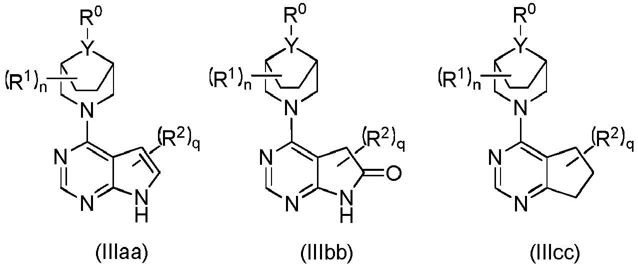 wherein:
Y, R⁰, R¹, R², q and n are defined as in general formula (I).

In some preferred embodiments of the present disclosure, provided are the compounds of general formulas (I), (IIaa), (IIaa-1), (IIaa-2), (Ilbb), (IIcc), (IIcc-1), (IIIaa), (IIIbb) and (IIIcc) or the tautomers, mesomers, racemates, enantiomers or diastereomers thereof or the mixtures thereof, or the pharmaceutically acceptable salts thereof, wherein R⁰ is-C(O)CHR⁵R⁶, and R⁵ and R⁶ are defined as in general formula (I).

In some embodiments of the present disclosure, provided are the compounds of general formulas (I), (IIaa), (IIaa-1), (IIaa-2), (Ilbb), (IIcc), (IIcc-1), (IIIaa), (IIIbb) and (IIIcc) or the tautomers, mesomers, racemates, enantiomers or diastereomers thereof or the mixtures thereof, or the pharmaceutically acceptable salts thereof, wherein R⁰ is - C(O)CHR⁵R⁶, wherein R⁵ is alkyl, wherein the alkyl is optionally substituted with one or more substituents selected from the group consisting of hydroxy and -NR⁹R¹⁰, and R⁹ and R¹⁰ are identical or different and are each independently a hydrogen atom or alkyl; R⁶ is aryl or heteroaryl, wherein the aryl or heteroaryl is optionally substituted with one or more substituents selected from the group consisting of halogen, alkyl, alkoxy, haloalkyl and haloalkoxy; preferably, R⁰ is 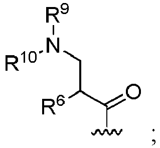 R⁶ is phenyl, wherein the phenyl is optionally substituted with one or more substituents selected from the group consisting of halogen, C₁₋₆ alkyl, C₁₋₆ alkoxy, C₁₋₆ haloalkyl and C₁₋₆ haloalkoxy; R⁹ and R¹⁰ are identical or different and are each independently a hydrogen atom or C₁₋₆ alkyl.

In some preferred embodiments of the present disclosure, provided is the compound of general formula (I) or the tautomer, mesomer, racemate, enantiomer or diastereomer thereof or the mixture thereof, or the pharmaceutically acceptable salt thereof, which is a compound of general formula (IV) or a tautomer, mesomer, racemate, enantiomer or diastereomer thereof or a mixture thereof, or a pharmaceutically acceptable salt thereof: 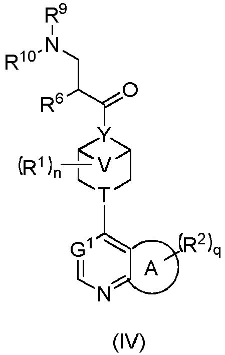 wherein:
R¹, R², R⁶, R⁹, R¹⁰, G¹, ring A, Y, V, T, q and n are defined as in general formula (I).

In some embodiments of the present disclosure, provided are the compounds of general formulas (I), (IIaa), (IIaa-1), (IIaa-2), (IIbb), (IIcc), (IIcc-1), (IIIaa), (IIIbb), (IIIcc) and (IV) or the tautomers, mesomers, racemates, enantiomers or diastereomers thereof or the mixtures thereof, or the pharmaceutically acceptable salts thereof, wherein Y is an N atom.

In some preferred embodiments of the present disclosure, provided is the compound of general formula (IV) or the tautomer, mesomer, racemate, enantiomer or diastereomer thereof or the mixture thereof, or the pharmaceutically acceptable salt thereof, wherein V is selected from the group consisting of -CH₂-, -C(CH₃)₂-, -CH₂CH₂-, -CH₂CH₂CH₂- and -CH₂OCH₂-.

In some preferred embodiments of the present disclosure, provided is the compound of general formula (IV) or the tautomer, mesomer, racemate, enantiomer or diastereomer thereof or the mixture thereof, or the pharmaceutically acceptable salt thereof, wherein T is an N atom.

In some preferred embodiments of the present disclosure, provided is the compound of general formula (IV) or the tautomer, mesomer, racemate, enantiomer or diastereomer thereof or the mixture thereof, or the pharmaceutically acceptable salt thereof, wherein Y is an N atom, T is an N atom, and V is selected from the group consisting of -CH₂CH₂-, - CH₂CH₂CH₂- and -CH₂OCH₂-; preferably, Y is an N atom, T is an N atom, and V is - CH₂CH₂-.

In some preferred embodiments of the present disclosure, provided is the compound of general formula (IV) or the tautomer, mesomer, racemate, enantiomer or diastereomer thereof or the mixture thereof, or the pharmaceutically acceptable salt thereof, wherein G¹ is CH or an N atom; preferably, G¹ is an N atom.

In some embodiments of the present disclosure, provided are the compounds of general formulas (I), (IIaa), (IIaa-1), (IIaa-2), (IIbb), (IIcc), (IIcc-1), (IIIaa), (IIIbb), (IIIcc) and (IV) or the tautomers, mesomers, racemates, enantiomers or diastereomers thereof or the mixtures thereof, or the pharmaceutically acceptable salts thereof, wherein R¹ is a hydrogen atom.

In some embodiments of the present disclosure, provided are the compounds of general formulas (I), (IIaa), (IIaa-1), (IIaa-2), (IIbb), (IIcc), (IIcc-1), (IIIaa), (IIIbb), (IIIcc) and (IV) or the tautomers, mesomers, racemates, enantiomers or diastereomers thereof or the mixtures thereof, or the pharmaceutically acceptable salts thereof, wherein R² are identical or different and are each independently selected from the group consisting of a hydrogen atom, oxo, halogen, alkyl, haloalkyl, hydroxyalkyl, hydroxy, alkoxy and haloalkoxy; preferably, R² are identical or different and are each independently selected from the group consisting of a hydrogen atom, oxo, halogen, C₁₋₆ alkyl, C₁₋₆ haloalkyl, C₁₋₆ hydroxyalkyl, hydroxy, C₁₋₆ alkoxy and C₁₋₆ haloalkoxy; more preferably, R² are identical or different and are each independently selected from the group consisting of a hydrogen atom, halogen, C₁₋₆ alkyl and hydroxy.

In some embodiments of the present disclosure, provided are the compounds of general formulas (I) and (IV) or the tautomers, mesomers, racemates, enantiomers or diastereomers thereof or the mixtures thereof, or the pharmaceutically acceptable salts thereof, wherein 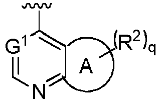 is selected from the group consisting of 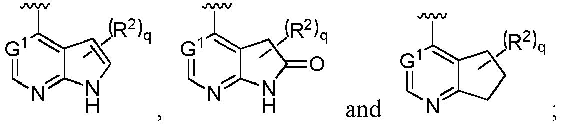 G¹, R² and q are defined as in general formula (I).

In some embodiments of the present disclosure, provided are the compounds of general formulas (I), (IIaa), (IIaa-1), (IIaa-2), (IIbb), (IIcc), (IIcc-1), (IIIaa), (IIIbb), (IIIcc) and (IV) or the tautomers, mesomers, racemates, enantiomers or diastereomers thereof or the mixtures thereof, or the pharmaceutically acceptable salts thereof, wherein n is 0, 1 or 2. In some embodiments of the present disclosure, provided are the compounds of general formulas (I), (IIcc), (IIIcc) and (IV) or the tautomers, mesomers, racemates, enantiomers or diastereomers thereof or the mixtures thereof, or the pharmaceutically acceptable salts thereof, wherein q is 0, 1, 2, 3, 4 or 5; provided are the compounds of general formulas (IIaa-1), (IIbb), (IIIaa) and (IIIbb) or the tautomers, mesomers, racemates, enantiomers or diastereomers thereof or the mixtures thereof, or the pharmaceutically acceptable salts thereof, wherein q is 0, 1, 2 or 3; provided are the compounds of general formulas (IIaa) and (IIaa-2) or the tautomers, mesomers, racemates, enantiomers or diastereomers thereof or the mixtures thereof, or the pharmaceutically acceptable salts thereof, wherein q is 0, 1 or 2.

In some embodiments of the present disclosure, provided are the compounds of general formulas (I), (IIaa), (IIaa-1), (IIaa-2), (IIbb), (IIcc), (IIIaa), (IIIbb), (IIIcc) and (IV) or the tautomers, mesomers, racemates, enantiomers or diastereomers thereof or the mixtures thereof, or the pharmaceutically acceptable salts thereof, wherein q is 0, 1 or 2. In some embodiments of the present disclosure, provided is the compound of general formula (IIbb) or (IIcc) or the pharmaceutically acceptable salt thereof, wherein:
Q is selected from the group consisting of groups of formulas (Qa) and (Qb): 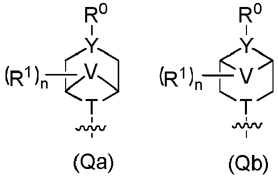
V is selected from the group consisting of -CH₂-, -C(CH₃)₂-, -CH₂CH₂-, -CH₂CH₂CH₂- and -CH₂OCH₂-; Y is an N atom; T is an N atom; R⁰ is 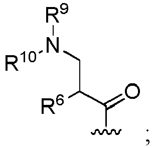 R⁶ is phenyl, wherein the phenyl is optionally substituted with one or more substituents selected from the group consisting of halogen, C₁₋₆ alkyl, C₁₋₆ alkoxy, C₁₋₆ haloalkyl and C₁₋₆ haloalkoxy; R⁹ and R¹⁰ are identical or different and are each independently a hydrogen atom or C₁₋₆ alkyl; R¹ is a hydrogen atom; G¹ is CH or an N atom; R² are identical or different and are each independently selected from the group consisting of a hydrogen atom, oxo, halogen, C₁₋₆ alkyl, C₁₋₆ haloalkyl, C₁₋₆ hydroxyalkyl, hydroxy, C₁₋₆ alkoxy and C₁₋₆ haloalkoxy; q is 0, 1 or 2.

In some embodiments of the present disclosure, provided is the compound of general formula (IIaa-1) or (IIaa-2) or the pharmaceutically acceptable salt thereof, wherein:
Q is selected from the group consisting of groups of formulas (Qa) and (Qb): 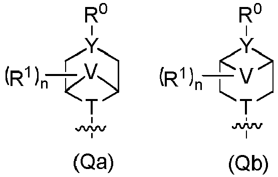
V is selected from the group consisting of -CH₂-, -C(CH₃)₂-, -CH₂CH₂-, -CH₂CH₂CH₂- and -CH₂OCH₂-; Y is an N atom; T is an N atom; R⁰ is 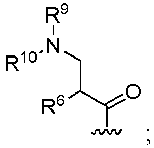 R⁶ is phenyl, wherein the phenyl is optionally substituted with one or more substituents selected from the group consisting of halogen, C₁₋₆ alkyl, C₁₋₆ alkoxy, C₁₋₆ haloalkyl and C₁₋₆ haloalkoxy; R⁹ and R¹⁰ are identical or different and are each independently a hydrogen atom or C₁₋₆ alkyl; R¹ is a hydrogen atom; G¹ is CH or an N atom; R² are identical or different and are each independently selected from the group consisting of a hydrogen atom, oxo, halogen, C₁₋₆ alkyl, C₁₋₆ haloalkyl, C₁₋₆ hydroxyalkyl, hydroxy, C₁₋₆ alkoxy and C₁₋₆ haloalkoxy; q is 0, 1 or 2.

**Table A. Typical compounds disclosed herein include, but are not limited to:**

| Example No. | Structure and name of compound |
|---|---|
| 1 | 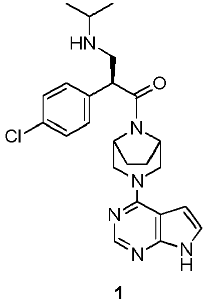 |
| | (*S*)-1-((1*R*,5*S*)-3-(7*H*-pyrrolo[2,3-*d*]pyrimidin-4-yl)-3,8-diazabicyclo[3.2.1]octan-8-yl)-2-(4-chlorophenyl)-3-(isopropylamino)propan-1-one **1** |
| 2 | 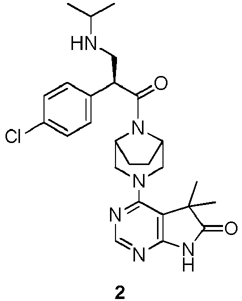 |
| | 4-((1*R*,5*S*)-8-(*S*)-2-(4-chlorophenyl)-3-(isopropylamino)propanoyl)-3,8-diazabicyclo[3.2.1]octan-3-yl)-5,5-dimethyl-5,7-dihydro-6*H-*pyrrolo[2,3-*d*]pyrimidin-6-one **2** |
| 3 | 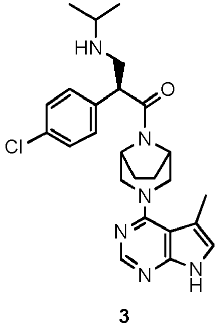 |
| | (*S*)-2-(4-chlorophenyl)-3-(isopropylamino)-1-((1*R*,5*S*)-3-(5-methyl-7*H*-pyrrolo[2,3-*d*]pyrimidin-4-yl)-3,8-diazabicyclo[3.2.1]octan-8-yl]propan-1-one **3** |
| 4 | 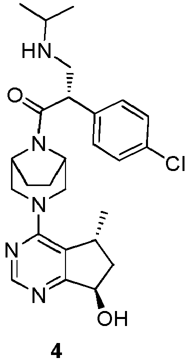 |
| | (*S*)-2-(4-chlorophenyl)-1-((1*R*,5*S*)-3-((5*R*,7*R*)-7-hydroxy-5-methyl-6,7-dihydro-5*H*-cyclopenta[*d*]pyrimidin-4-yl)-3,8-diazabicyclo[3.2.1]octan-8-yl)-3-(isopropylamino)propan- 1 -one **4** |
| 5 | 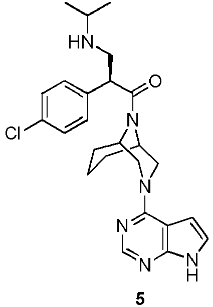 |
| | (2*S*)-1-(3-(7*H*-pyrrolo[2,3-*d*]pyrimidin-4-yl)-3,9-diazabicyclo[3.3.1]nonan-9-yl)-2-(4-chlorophenyl)-3-(isopropylamino)propan-1-one **5** |
| 6 | 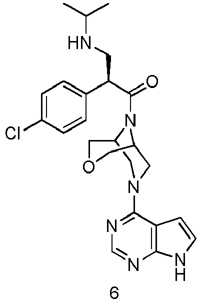 |
| | (2*S*)-1-(7-(7*H*-pyrrolo[2,3-*d*]pyrimidin-4-yl)-3-oxa-7,9-diazabicyclo[3.3.1]nonan-9-yl)-2-(4-chlorophenyl)-3-(isopropylamino)propan-1-one **6** |
| 7 | 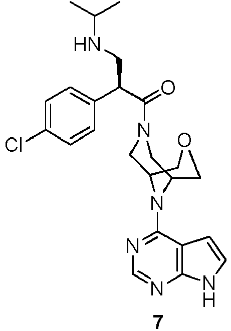 |
| | (2*S*)-1-(9-(7*H*-pyrrolo[2,3-*d*]pyrimidin-4-yl)-3-oxa-7,9-diazabicyclo[3.3.1]nonan-7-yl)-2-(4-chlorophenyl)-3-(isopropylamino)-propan-1-one **7** |
| 8 | 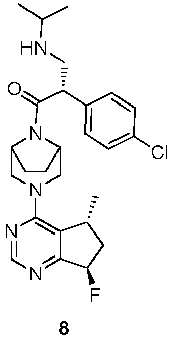 |
| | (*S*)-2-(4-chlorophenyl)-1-((1*R*,5*S*)-3-((5*R*,7*R*)-7-fluoro-5-methyl-6,7-dihydro-5*H*-cyclopenta[*d*]pyrimidin-4-yl)-3,8-diazabicyclo[3.2.1]octan-8-yl)-3-(isopropylamino)propan-1-one **8** |
| 9 | 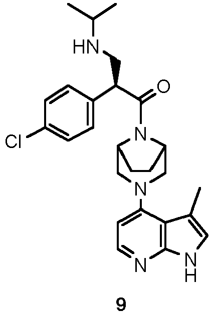 |
| | (*S*)-2-(4-chlorophenyl)-3-(isopropylamino)-1-((1*R*,5*S*)-3-(3-methyl-1*H*-pyrrolo[2,3-*b*]pyridin-4-yl)-3,8-diazabicyclo[3.2.1]octan-8-yl)propan-1-one **9** |

Another aspect of the present disclosure relates to a compound of general formula (IVA) or a tautomer, mesomer, racemate, enantiomer or diastereomer thereof or a mixture thereof, or a pharmaceutically acceptable salt thereof, 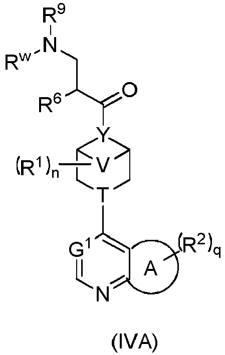 wherein:
R^{w} is an amino protecting group; and
R¹, R², R⁶, R⁹, G¹, ring A, Y, V, T, n and q are defined as in general formula (IV). Another aspect of the present disclosure relates to the compound of general formula (IVA) or the tautomer, mesomer, racemate, enantiomer or diastereomer thereof or the mixture thereof, or the pharmaceutically acceptable salt thereof, wherein R^{w} is *tert-*butoxycarbonyl.

In some embodiments of the present disclosure, provided is the compound of general formula (IVA) or the tautomer, mesomer, racemate, enantiomer, diastereomer or the mixture thereof, or the pharmaceutically acceptable salt thereof, wherein 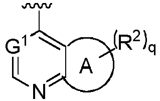 is selected from the group consisting of 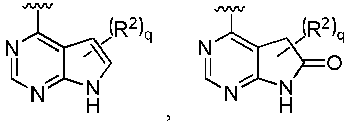 and 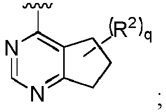 R² and q are defined as in general formula (IV); preferably, q is 0, 1 or 2.

Typical intermediate compounds disclosed herein include, but are not limited to:

| Example No. | Structure and name of compound |
|---|---|
| 1f | 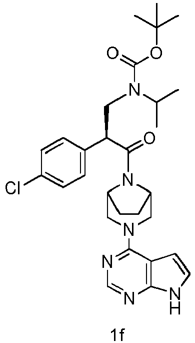 |
| | Tert-butyl ((*S*)-(3-((1*R*,5*S*)-3-(7*H*-pyrrolo[2,3-*d*]pyrimidin-4-yl)-3,8-diazabicyclo[3.2.1]octan-8-yl)-2-(4-chlorophenyl)-3-oxopropyl)(isopropyl)carbamate **1f** |
| 2d | 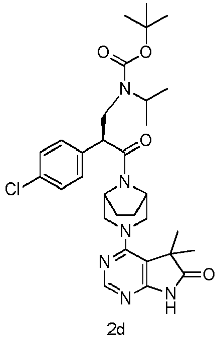 |
| | *Tert*-butyl ((*S*)-2-(4-chlorophenyl)-3-((1*R*,5*S*)-3-(5,5-dimethyl-6-oxo-6,7-dihydro-5*H*-pyrrolo[2,3-*d*]pyrimidin-4-yl)-3,8-diazabicyclo[3.2.1]octan-8-yl)-3-oxopropyl)(isopropyl)carbamate **2d** |
| 3d | 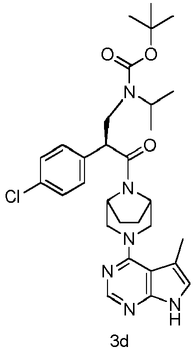 |
| | *Tert*-butyl ((*S*)-2-(4-chlorophenyl)-3-((1*R*,5*S*)-3-(5-methyl-7*H-*pyrrolo[2,3-*d*]pyrimidin-4-yl)-3,8-diazabicyclo[3.2.1]octan-8-yl)-3-oxopropyl)(isopropyl)carbamate **3d** |
| 4e | 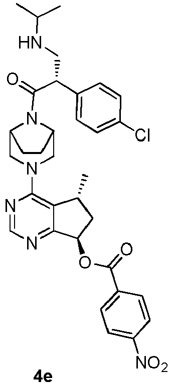 |
| | (5*R*,7*R*)-4-((1*R*,5*S*)-8-((*S*)-2-(4-chlorophenyl)-3-(isopropylamino)propanoyl)-3,8-diazabicyclo[3.2.1]octan-3-yl)-5-methyl-6,7-dihydro-5*H*-cyclopenta[*d*]pyrimidin-7-yl 4-nitrobenzoate **4e** |
| 5d | 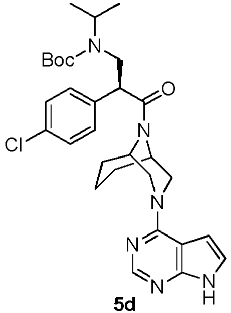 |
| | *Tert*-butyl ((2*S*)-3-(3-(7*H*-pyrrolo[2,3-*d*]pyrimidin-4-yl)-3,9-diazabicyclo[3.3.1]nonan-9-yl)-2-(4-chlorophenyl)-3-oxopropyl)(isopropyl)carbamate **5d** |
| 8e | 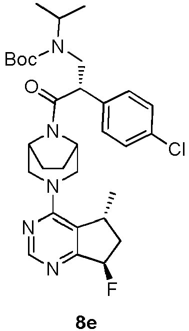 |
| | *Tert*-butyl ((*S*)-2-(4-chlorophenyl)-3-((1*R*,5*S*)-3-((5*R*,7*R*)-7-fluoro-5-methyl-6,7-dihydro-5*H*-cyclopenta[*d*]pyrimidin-4-yl)-3,8-diazabicyclo[3.2.1]octan-8-yl)-3-oxopropyl)(isopropyl)carbamate **8e** |
| 9e | 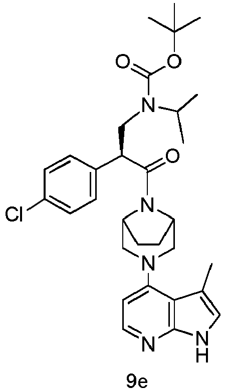 |
| | *Tert*-butyl ((*S*)-2-(4-chlorophenyl)-3-((1*R*,5*S*)-3-(3-methyl-1*H-*pyrrolo[2,3-*b*]pyridin-4-yl)-3,8-diazabicyclo[3.2.1]octan-8-yl)-3-oxopropyl)(isopropyl)carbamate **9e** |

The Boc is *tert*-butyloxycarbonyl.

Another aspect of the present disclosure relates to a compound of general formula (IIcc-1A) or a tautomer, mesomer, racemate, enantiomer or diastereomer thereof or a mixture thereof, or a pharmaceutically acceptable salt thereof, 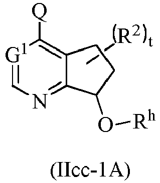 wherein:
R^{h} is a hydroxy protecting group, preferably *p*-nitrobenzoyl; and
G¹, Q, R² and t are defined as in the compound of general formula (IIcc-1).

Another aspect of the present disclosure relates to a method for preparing the compound of general formula (IIcc-1) or the tautomer, mesomer, racemate, enantiomer or diastereomer thereof or the mixture thereof, or the pharmaceutically acceptable salt thereof, which comprises: 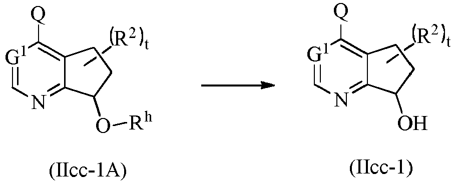 removing a hydroxy protecting group R^{h} from a compound of general formula (IIcc-1A) to obtain the compound of general formula (IIcc-1), wherein:
R^{h} is the hydroxy protecting group, preferably *p*-nitrobenzoyl; and
G¹, Q, R² and t are defined as in the compound of general formula (IIcc-1).

Another aspect of the present disclosure relates to a method for preparing the compound of general formula (IV) or the tautomer, mesomer, racemate, enantiomer or diastereomer thereof or the mixture thereof, or the pharmaceutically acceptable salt thereof, which comprises: 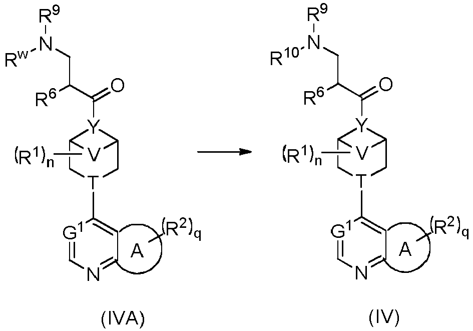 removing an amino protecting group from a compound of general formula (IVA) to obtain the compound of general formula (IV), wherein:
R^{w} is an amino protecting group;
R¹⁰ is a hydrogen atom; and
R¹, R², R⁶, R⁹, Y, V, T, ring A, G¹, n and q are defined as in general formula (IV).

Another aspect of the present disclosure relates to a pharmaceutical composition comprising the compounds of general formulas (I), (IIaa), (IIaa-1), (IIaa-2), (IIbb), (IIcc), (IIcc-1), (IIIaa), (IIIbb), (IIIcc) and (IV) and the compounds shown in Table A, or the tautomers, mesomers, racemates, enantiomers or diastereomers thereof or the mixtures thereof, or the pharmaceutically acceptable salts thereof, and one or more pharmaceutically acceptable carriers, diluents or excipients.

The present disclosure further relates to use of the compounds of general formulas (I), (IIaa), (IIaa-1), (IIaa-2), (IIbb), (IIcc), (IIcc-1), (IIIaa), (IIIbb), (IIIcc) and (IV) and the compounds shown in Table A, or the tautomers, mesomers, racemates, enantiomers or diastereomers thereof or the mixtures thereof, or the pharmaceutically acceptable salts thereof, or the pharmaceutical composition comprising the same in preparing a medicament for inhibiting AKT1/2/3(AKT pan).

The present disclosure further relates to use of the compounds of general formulas (I), (IIaa), (IIaa-1), (IIaa-2), (IIbb), (IIcc), (IIcc-1), (IIIaa), (IIIbb), (IIIcc) and (IV) and the compounds shown in Table A, or the tautomers, mesomers, racemates, enantiomers or diastereomers thereof or the mixtures thereof, or the pharmaceutically acceptable salts thereof, or the pharmaceutical composition comprising the same in preparing a medicament for treating and/or preventing a tumor, preferably in preparing a medicament for treating and/or preventing cancer.

The present disclosure further relates to use of the compounds of general formulas (I), (IIaa), (IIaa-1), (IIaa-2), (IIbb), (IIcc), (IIcc-1), (IIIaa), (IIIbb), (IIIcc) and (IV) and the compounds shown in Table A, or the tautomers, mesomers, racemates, enantiomers or diastereomers thereof or the mixtures thereof, or the pharmaceutically acceptable salts thereof, or the pharmaceutical composition comprising the same in preparing a medicament for treating and/or preventing a disease or disorder, wherein the disease or disorder is selected from the group consisting of ovarian cancer, breast cancer, prostate cancer, neuroglioma, glioblastoma, gastric cancer, fallopian tube cancer, lung cancer, peritoneal tumor, melanoma, brain cancer, esophageal cancer, liver cancer, pancreatic cancer, colorectal cancer, lung cancer, kidney cancer, cervical cancer, skin cancer, neuroblastoma, sarcoma, bone cancer, uterine cancer, endometrial cancer, head and neck tumor, multiple myeloma, lymphoma, non-Hodgkin's lymphoma, non-small cell lung cancer, polycythemia vera, leukemia, thyroid tumor, bladder cancer and gallbladder cancer.

The present disclosure also relates to a method for inhibiting AKT 1/2/3 (AKT pan), which comprises administering to a patient in need thereof a therapeutically effective amount of the compounds of general formulas (I), (IIaa), (IIaa-1), (IIaa-2), (Ilbb), (IIcc), (IIcc-1), (IIIaa), (IIIbb), (IIIcc) and (IV) and the compounds shown in Table A, or the tautomers, mesomers, racemates, enantiomers or diastereomers thereof or the mixtures thereof, or the pharmaceutically acceptable salts thereof, or the pharmaceutical composition comprising the same.

The present disclosure also relates to a method for treating and/or preventing a tumor, preferably a method for treating and/or preventing cancer, which comprises administering to a patient in need thereof a therapeutically effective amount of the compounds of general formulas (I), (IIaa), (IIaa-1), (IIaa-2), (IIbb), (IIcc), (IIcc-1), (IIIaa), (IIIbb), (IIIcc) and (IV) and the compounds shown in Table A, or the tautomers, mesomers, racemates, enantiomers or diastereomers thereof or the mixtures thereof, or the pharmaceutically acceptable salts thereof, or the pharmaceutical composition comprising the same.

The present disclosure also relates to a method for treating and/or preventing a disease or disorder, which comprises administering to a patient in need thereof a therapeutically effective amount of the compounds of general formulas (I), (IIaa), (IIaa-1), (IIaa-2), (IIbb), (IIcc), (IIcc-1), (IIIaa), (IIIbb), (IIIcc) and (IV) and the compounds shown in Table A, or the tautomers, mesomers, racemates, enantiomers or diastereomers thereof or the mixtures thereof, or the pharmaceutically acceptable salts thereof, or the pharmaceutical composition comprising the same, wherein the disease or disorder is preferably selected from the group consisting of ovarian cancer, breast cancer, prostate cancer, neuroglioma, glioblastoma, gastric cancer, fallopian tube cancer, lung cancer, peritoneal tumor, melanoma, brain cancer, esophageal cancer, liver cancer, pancreatic cancer, colorectal cancer, lung cancer, kidney cancer, cervical cancer, skin cancer, neuroblastoma, sarcoma, bone cancer, uterine cancer, endometrial cancer, head and neck tumor, multiple myeloma, lymphoma, non-Hodgkin's lymphoma, non-small cell lung cancer, polycythemia vera, leukemia, thyroid tumor, bladder cancer and gallbladder cancer.

The present disclosure further relates to the compounds of general formulas (I), (IIaa), (IIaa-1), (IIaa-2), (IIbb), (IIcc), (IIcc-1), (IIIaa), (IIIbb), (IIIcc) and (IV) and the compounds shown in Table A, or the tautomers, mesomers, racemates, enantiomers or diastereomers thereof or the mixtures thereof, or the pharmaceutically acceptable salts thereof, or the pharmaceutical composition comprising the same for use as a medicament. The present disclosure also relates to the compounds of general formulas (I), (IIaa), (IIaa-1), (IIaa-2), (IIbb), (IIcc), (IIcc-1), (IIIaa), (IIIbb), (IIIcc) and (IV) and the compounds shown in Table A, or the tautomers, mesomers, racemates, enantiomers or diastereomers thereof or the mixtures thereof, or the pharmaceutically acceptable salts thereof, or the pharmaceutical composition comprising the same for use as an AKT1/2/3(AKT pan) inhibitor.

The present disclosure further relates to the compounds of general formulas (I), (IIaa), (IIaa-1), (IIaa-2), (IIbb), (IIcc), (IIcc-1), (IIIaa), (IIIbb), (IIIcc) and (IV) and the compounds shown in Table A, or the tautomers, mesomers, racemates, enantiomers or diastereomers thereof or the mixtures thereof, or the pharmaceutically acceptable salts thereof, or the pharmaceutical composition comprising the same for use in treating a tumor, preferably in treating cancer.

The tumor described herein is selected from the group consisting of melanoma, brain tumor, esophageal cancer, gastric cancer, liver cancer, pancreatic cancer, colorectal cancer, lung cancer, kidney cancer, breast cancer, cervical cancer, ovarian cancer, prostate cancer, skin cancer, neuroblastoma, neuroglioma, glioblastoma, sarcoma, bone cancer, uterine cancer, endometrial cancer, head and neck tumor, multiple myeloma, B-cell lymphoma, polycythemia vera, leukemia, thyroid tumor, bladder cancer and gallbladder cancer.

The present disclosure further relates to the compounds of general formulas (I), (IIaa), (IIaa-1), (IIaa-2), (IIbb), (IIcc), (IIcc-1), (IIIaa), (IIIbb), (IIIcc) and (IV) and the compounds shown in Table A, or the tautomers, mesomers, racemates, enantiomers or diastereomers thereof or the mixtures thereof, or the pharmaceutically acceptable salts thereof, or the pharmaceutical composition comprising the same for use in treating a disease or disorder, wherein the disease or disorder is selected from the group consisting of ovarian cancer, breast cancer, prostate cancer, neuroglioma, glioblastoma, gastric cancer, fallopian tube cancer, lung cancer, peritoneal tumor, melanoma, brain cancer, esophageal cancer, liver cancer, pancreatic cancer, colorectal cancer, lung cancer, kidney cancer, cervical cancer, skin cancer, neuroblastoma, sarcoma, bone cancer, uterine cancer, endometrial cancer, head and neck tumor, multiple myeloma, lymphoma, non-Hodgkin's lymphoma, non-small cell lung cancer, polycythemia vera, leukemia, thyroid tumor, bladder cancer and gallbladder cancer.

The tumor, cancer, disease or disorder described above is preferably AKT1/2/3-mediated tumor, cancer, disease or disorder.

The active compounds may be formulated into a form suitable for administration by any suitable route, and one or more pharmaceutically acceptable carriers are used to formulate the compositions of the present disclosure by conventional methods. Thus, the active compounds of the present disclosure may be formulated into a variety of dosage forms for oral administration, administration by injection (e.g., intravenous, intramuscular or subcutaneous), or administration by inhalation or insufflation. The compounds of the present disclosure may also be formulated into a sustained-release dosage form, such as tablets, hard or soft capsules, aqueous or oily suspensions, emulsions, injections, dispersible powders or granules, suppositories, lozenges or syrups.

The dose of the compound or composition used in the treatment method of the present disclosure will generally vary with the severity of the disease, the body weight of the patient, and the relative efficacy of the compound. However, as a general guide, the active compound is preferably in a form of a unit dose, or in a form of a single dose that can be self-administered by a patient. The unit dose of the compound or composition of the present disclosure may be in a tablet, capsule, cachet, vial, powder, granule, lozenge, suppository, regenerating powder or liquid formulation. A suitable unit dose may be 0.1-1000 mg.

The pharmaceutical composition of the present disclosure may comprise, in addition to the active compound, one or more auxiliary materials selected from the group consisting of a filler (diluent), a binder, a wetting agent, a disintegrant, an excipient, and the like. Depending on the method of administration, the compositions may comprise 0.1 to 99 wt.% of the active compound.

The tablet comprises the active ingredient and a non-toxic pharmaceutically acceptable excipient that is used for mixing and is suitable for the preparation of the tablet. Such an excipient may be an inert excipient, a granulating agent, a disintegrating agent, a binder and a lubricant. Such a tablet may be uncoated or may be coated by known techniques for masking the taste of the drug or delaying the disintegration and absorption of the drug in the gastrointestinal tract and thus enabling sustained release of the drug over a longer period.

An oral formulation in a soft gelatin capsule where the active ingredient is mixed with an inert solid diluent or with a water-soluble carrier or oil vehicle may also be provided. An aqueous suspension comprises an active substance and an excipient that is used for mixing and suitable for the preparation of the aqueous suspension. Such an excipient is a suspending agent, a dispersant or a wetting agent. The aqueous suspension may also comprise one or more preservatives, one or more colorants, one or more corrigents and one or more sweeteners.

An oil suspension may be formulated by suspending the active ingredient in a vegetable oil, or in a mineral oil. The oil suspension may comprise a thickening agent. The sweeteners and corrigents described above may be added to provide a palatable formulation. Antioxidants may also be added to preserve the compositions.

The pharmaceutical composition disclosed herein may also be in the form of an oil-in-water emulsion. The oil phase may be a vegetable oil or a mineral oil, or a mixture thereof. Suitable emulsifiers may be naturally occurring phospholipids, and the emulsion may also comprise a sweetener, a corrigent, a preservative and an antioxidant. Such a formulation may also comprise a palliative, a preservative, a colorant and an antioxidant.

The pharmaceutical composition disclosed herein may be in a form of a sterile injectable aqueous solution. Available and acceptable vehicles or solvents include water, Ringer's solution and isotonic sodium chloride solution. A sterile injectable formulation may be a sterile injectable oil-in-water microemulsion in which an active ingredient is dissolved in an oil phase. The injection or microemulsion can be locally injected into the bloodstream of a patient in large quantities. Alternatively, it may be desirable to administer solutions and microemulsions in such a way as to maintain a constant circulating concentration of the compound disclosed herein. To maintain such a constant concentration, a continuous intravenous delivery device may be used. An example of such a device is a Deltec CADD-PLUS. TM. 5400 intravenous injection pump.

The pharmaceutical composition disclosed herein may be in a form of a sterile injectable aqueous or oil suspension for intramuscular and subcutaneous administration. The suspension can be prepared according to the prior art using those suitable dispersants or wetting agents and suspending agents mentioned above. The sterile injectable formulation may also be a sterile injection or suspension prepared in a parenterally acceptable non-toxic diluent or solvent. In addition, a sterile fixed oil may be conventionally used as a solvent or a suspending medium. For this purpose, any blend fixed oil may be employed. In addition, fatty acids may also be used to prepare injections.

The compound of the present disclosure may be administered in the form of a suppository for rectal administration. Such a pharmaceutical composition can be prepared by mixing a drug with a suitable non-irritating excipient which is a solid at ambient temperature but a liquid in the rectum and therefore will melt in the rectum to release the drug.

The compounds of the present disclosure can be administered in the form of dispersible powders and granules that are formulated into aqueous suspensions by adding water. These pharmaceutical compositions can be prepared by mixing the active ingredient with a dispersant or a wetting agent, a suspending agent, or one or more preservatives.

As is well known to those skilled in the art, the dose of the drug administered depends on a variety of factors, including but not limited to, the activity of the particular compound employed, the age of the patient, the weight of the patient, the health condition of the patient, the behavior of the patient, the diet of the patient, the time of administration, the route of administration, the rate of excretion, the combination of drugs, and the like. In addition, the optimal treatment regimen, such as the mode of administration, the daily dose of the compound or the type of pharmaceutically acceptable salts, can be verified according to conventional treatment regimens.

### Definition of terms

Unless otherwise stated, the terms used in the specification and claims have the following meanings.

The term "alkyl" refers to a saturated aliphatic hydrocarbon group which is a linear or branched group containing 1 to 20 carbon atoms, preferably alkyl containing 1 to 12 (e.g., 1, 2, 3, 4, 5, 6, 7, 8, 9, 10, 11 and 12) carbon atoms, and more preferably alkyl containing 1 to 6 carbon atoms. Non-limiting examples include methyl, ethyl, *n*-propyl, isopropyl, *n*-butyl, isobutyl, *tert*-butyl, *sec*-butyl, *n*-pentyl, 1,1-dimethylpropyl, 1,2-dimethylpropyl, 2,2-dimethylpropyl, 1-ethylpropyl, 2-methylbutyl, 3-methylbutyl, *n*-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, *n*-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, *n*-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl, *n*-nonyl, 2-methyl-2-ethylhexyl, 2-methyl-3-ethylhexyl, 2,2-diethylpentyl, *n*-decyl, 3,3-diethylhexyl, 2,2-diethylhexyl, and various side-chain isomers thereof, etc. More preferred is a lower alkyl having 1 to 6 carbon atoms, and non-limiting examples include methyl, ethyl, *n*-propyl, isopropyl, *n*-butyl, isobutyl, *tert*-butyl, *sec*-butyl, *n*-pentyl, 1,1-dimethylpropyl, 1,2-dimethylpropyl, 2,2-dimethylpropyl, 1-ethylpropyl, 2-methylbutyl, 3-methylbutyl, *n*-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl and the like. The alkyl may be substituted or unsubstituted. When substituted, the substituent may be substituted at any available connection site, and the substituent is preferably one or more substituents independently optionally selected from the group consisting of a D atom, halogen, alkyl, alkoxy, haloalkyl, haloalkoxy, cycloalkyloxy, heterocyclyloxy, hydroxy, hydroxyalkyl, cyano, amino, nitro, cycloalkyl, heterocyclyl, aryl and heteroaryl.

The term "alkoxy" refers to -O-(alkyl), wherein the alkyl is defined as above. Non-limiting examples of alkoxy include methoxy, ethoxy, propoxy and butoxy. Alkoxy may be optionally substituted or unsubstituted. When substituted, the substituent is preferably one or more groups independently selected from the group consisting of a D atom, halogen, alkoxy, haloalkyl, haloalkoxy, cycloalkyloxy, heterocyclyloxy, hydroxy, hydroxyalkyl, cyano, amino, nitro, cycloalkyl, heterocyclyl, aryl and heteroaryl.

The term "alkylene" refers to a saturated linear or branched aliphatic hydrocarbon group, which is a residue derived from the parent alkane by removal of two hydrogen atoms from the same carbon atom or two different carbon atoms. It is a linear or branched group containing 1 to 20 carbon atoms, preferably alkylene containing 1 to 12 (e.g., 1, 2, 3, 4, 5, 6, 7, 8, 9, 10, 11 and 12) carbon atoms, more preferably alkylene containing 1 to 6 carbon atoms. Non-limiting examples of alkylene include, but are not limited to, methylene (-CH₂-), 1,1-ethylene (-CH(CH₃)-), 1,2-ethylene (-CH₂CH₂-), 1,1-propylene (-CH(CH₂CH₃)-), 1,2-propylene (-CH₂CH(CH₃)-), 1,3-propylene (-CH₂CH₂CH₂-), 1,4-butylene (-CH₂CH₂CH₂CH₂-), etc. The alkylene may be substituted or unsubstituted. When substituted, the substituent may be substituted at any available connection site, and the substituent is preferably one or more substituents independently optionally selected from the group consisting of alkenyl, alkynyl, alkoxy, haloalkoxy, cycloalkyloxy, heterocyclyloxy, alkylthio, alkylamino, halogen, mercapto, hydroxy, nitro, cyano, cycloalkyl, heterocyclyl, aryl, heteroaryl, cycloalkoxy, heterocycloalkoxy, cycloalkylthio, heterocycloalkylthio and oxo.

The term "alkenyl" refers to an alkyl compound containing at least one carbon-carbon double bond in the molecule, wherein the alkyl is defined as above. The alkenyl is preferably one containing 2 to 12 (e.g., 2, 3, 4, 5, 6, 7, 8, 9, 10, 11 and 12) carbon atoms and more preferably one containing 2 to 6 carbon atoms. Alkenyl may be substituted or unsubstituted. When substituted, the substituent is preferably one or more groups independently selected from the group consisting of alkoxy, halogen, haloalkyl, haloalkoxy, cycloalkyloxy, heterocyclyloxy, hydroxy, hydroxyalkyl, cyano, amino, nitro, cycloalkyl, heterocyclyl, aryl and heteroaryl.

The term "alkynyl" refers to an alkyl compound containing at least one carbon-carbon triple bond in the molecule, wherein the alkyl is defined as above. The alkynyl is preferably one containing 2 to 12 (e.g., 2, 3, 4, 5, 6, 7, 8, 9, 10, 11 and 12) carbon atoms and more preferably one containing 2 to 6 carbon atoms. Alkynyl may be substituted or unsubstituted. When substituted, the substituent is preferably one or more groups independently selected from the group consisting of alkoxy, halogen, haloalkyl, haloalkoxy, cycloalkyloxy, heterocyclyloxy, hydroxy, hydroxyalkyl, cyano, amino, nitro, cycloalkyl, heterocyclyl, aryl and heteroaryl.

The term "cycloalkyl" refers to a saturated or partially unsaturated monocyclic or polycyclic hydrocarbon substituent. The cycloalkyl ring contains 3 to 20 carbon atoms, preferably 3 to 12 (e.g., 3, 4, 5, 6, 7, 8, 9, 10, 11 and 12) carbon atoms, preferably 3 to 8 (e.g., 3, 4, 5, 6, 7 and 8) carbon atoms, and more preferably 3 to 6 carbon atoms. Non-limiting examples of monocyclic cycloalkyl include cyclopropyl, cyclobutyl, cyclopentyl, cyclopentenyl, cyclohexyl, cyclohexenyl, cyclohexadienyl, cycloheptyl, cycloheptatrienyl, cyclooctyl, and the like. Polycyclic cycloalkyl includes spiro cycloalkyl, fused cycloalkyl, and bridged cycloalkyl.

The term "spiro cycloalkyl" refers to a 5-20 membered polycyclic group in which monocyclic rings share one carbon atom (referred to as the spiro atom), wherein the spiro cycloalkyl may contain one or more double bonds. The spiro cycloalkyl is preferably 6-14 (e.g., 6, 7, 8, 9, 10, 11, 12, 13 and 14) membered and more preferably 7-10 (e.g., 7, 8,

9 or 10) membered. According to the number of the spiro atoms shared among the rings, the spiro cycloalkyl may be monospiro cycloalkyl, bispiro cycloalkyl or polyspiro cycloalkyl, preferably monospiro cycloalkyl and bispiro cycloalkyl, more preferably 3-membered/4-membered, 3-membered/5-membered, 3-membered/6-membered, 4-membered/4-membered, 4-membered/5-membered, 4-membered/6-membered, 5-membered/4-membered, 5-membered/5-membered, 5-membered/6-membered, 6-membered/3-membered, 6-membered/4-membered, 6-membered/5-membered and 6-membered/6-membered monospiro cycloalkyl. Non-limiting examples of spiro cycloalkyl include:

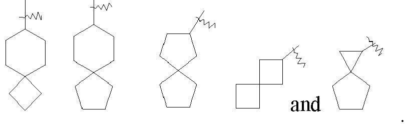

The term "fused cycloalkyl" refers to a 5-20 membered carbon polycyclic group in which each ring shares a pair of adjacent carbon atoms with the other rings in the system, wherein one or more of the rings may contain one or more double bonds. The fused cycloalkyl is preferably 6-14 (e.g., 6, 7, 8, 9, 10, 11, 12, 13 and 14) membered and more preferably 7-10 (e.g., 7, 8, 9 or 10) membered. According to the number of the formed rings, the fused cycloalkyl may be bicyclic, tricyclic, tetracyclic or polycyclic cycloalkyl, preferably bicyclic or tricyclic cycloalkyl, and more preferably 3-membered/4-membered, 3-membered/5-membered, 3-membered/6-membered, 4-membered/4-membered, 4-membered/5-membered, 4-membered/6-membered, 5-membered/4-membered, 5-membered/5-membered, 5-membered/6-membered, 6-membered/3-membered, 6-membered/4-membered, 6-membered/5-membered and 6-membered/6-membered bicyclic cycloalkyl. Non-limiting examples of fused cycloalkyl include:

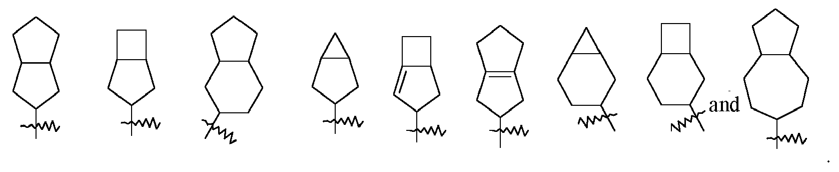

The term "bridged cycloalkyl" refers to a 5-20 membered carbon polycyclic group in which any two rings share two carbon atoms that are not directly connected to each other, wherein the bridged cycloalkyl may contain one or more double bonds. The bridged cycloalkyl is preferably 6-14 (e.g., 6, 7, 8, 9, 10, 11, 12, 13 and 14) membered and more preferably 7-10 (e.g., 7, 8, 9 or 10) membered. According to the number of the formed rings, the bridged cycloalkyl may be bicyclic, tricyclic, tetracyclic or polycyclic, preferably bicyclic, tricyclic or tetracyclic, and more preferably bicyclic or tricyclic. Non-limiting examples of bridged cycloalkyl include:

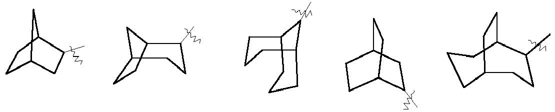

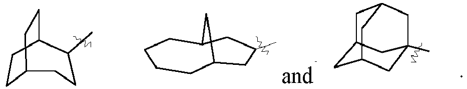

The cycloalkyl ring includes those in which the cycloalkyl described above (including monocyclic, spiro, fused and bridged rings) is fused to an aryl, heteroaryl or heterocycloalkyl ring, wherein the ring connected to the parent structure is cycloalkyl. Non-limiting examples include indanyl, tetrahydronaphthyl, benzocycloheptanyl, and the like, and preferably indanyl and tetrahydronaphthyl.

The cycloalkyl may be substituted or unsubstituted. When substituted, the substituent may be substituted at any available connection site, and the substituent is preferably one or more substituents independently optionally selected from the group consisting of halogen, alkyl, alkoxy, haloalkyl, haloalkoxy, cycloalkyloxy, heterocyclyloxy, hydroxy, hydroxyalkyl, cyano, amino, nitro, cycloalkyl, heterocyclyl, aryl and heteroaryl.

The term "heterocyclyl" refers to a saturated or partially unsaturated monocyclic or polycyclic hydrocarbon substituent containing 3 to 20 ring atoms, wherein one or more of the ring atoms are heteroatoms selected from the group consisting of nitrogen, oxygen, sulfur, S(O) and S(O)₂, excluding a cyclic portion of -O-O-, -O-S- or -S-S-, and the remaining ring atoms are carbon atoms. The heterocyclyl preferably contains 3 to 12 (e.g., 3, 4, 5, 6, 7, 8, 9, 10, 11 and 12) ring atoms, of which 1 to 4 (e.g., 1, 2, 3 and 4) are heteroatoms; more preferably 3 to 8 (e.g., 3, 4, 5, 6, 7 and 8) ring atoms, of which 1 to 3 (e.g., 1, 2 and 3) are heteroatoms; more preferably 3 to 6 ring atoms, of which 1 to 3 are heteroatoms; most preferably 5 or 6 ring atoms, of which 1 to 3 are heteroatoms. Non-limiting examples of monocyclic heterocyclyl include pyrrolidinyl, tetrahydropyranyl, 1,2.3.6-tetrahydropyridinyl, piperidinyl, piperazinyl, morpholinyl, thiomorpholinyl, homopiperazinyl, and the like. Non-limiting examples of polycyclic heterocyclyl include spiro heterocyclyl, fused heterocyclyl, and bridged heterocyclyl.

The term "spiro heterocyclyl" refers to a 5-20 membered polycyclic heterocyclyl group in which monocyclic rings share one atom (referred to as the spiro atom), wherein one or more ring atoms are heteroatoms selected from the group consisting of nitrogen, oxygen, sulfur, S(O) and S(O)₂, and the remaining ring atoms are carbon atoms. The spiro heterocyclyl may contain one or more double bonds. The spiro heterocyclyl is preferably 6-14 (e.g., 6, 7, 8, 9, 10, 11, 12, 13 and 14) membered and more preferably 7-10 (e.g., 7, 8, 9 or 10) membered. According to the number of spiro atoms shared among the rings, the spiro heterocyclyl may be monospiro heterocyclyl, bispiro heterocyclyl or polyspiro heterocyclyl, preferably monospiro heterocyclyl and bispiro heterocyclyl, more preferably 3-membered/4-membered, 3-membered/5-membered, 3-membered/6-membered, 4-membered/4-membered, 4-membered/5-membered, 4-membered/6-membered, 5-membered/4-membered, 5-membered/5-membered, 5-membered/6-membered, 6-membered/3-membered, 6-membered/4-membered, 6-membered/5-membered and 6-membered/6-membered monospiro heterocyclyl. Non-limiting examples of spiro heterocyclyl include:

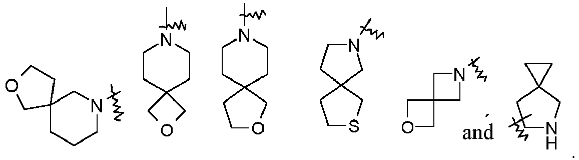

The term "fused heterocyclyl" refers to a 5-20 membered polycyclic heterocyclyl group in which each ring shares a pair of adjacent atoms with the other rings in the system, wherein one or more of the rings may contain one or more double bonds. In the fused heterocyclyl, one or more of the ring atoms are heteroatoms selected from the group consisting of nitrogen, oxygen, sulfur, S(O) and S(O)₂, and the remaining ring atoms are carbon atoms. The fused heterocyclyl is preferably 6-14 (e.g., 6, 7, 8, 9, 10, 11, 12, 13 and 14) membered and more preferably 7-10 (e.g., 7, 8, 9 or 10) membered. According to the number of the formed rings, the fused heterocyclyl may be bicyclic, tricyclic, tetracyclic or polycyclic fused heterocyclyl, preferably bicyclic or tricyclic fused heterocyclyl, and more preferably 5-membered/5-membered or 5-membered/6-membered bicyclic fused heterocyclyl. Non-limiting examples of fused heterocyclyl include:

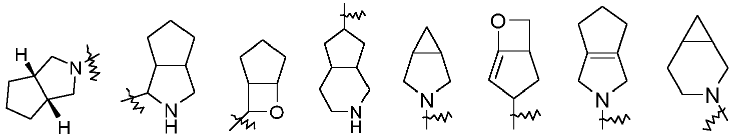

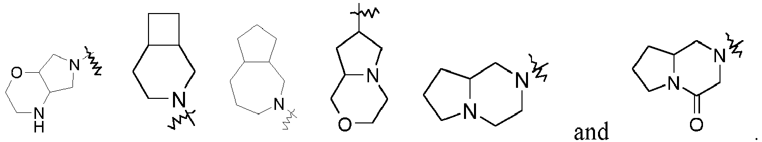

The term "bridged heterocyclyl" refers to a 5-14 membered polycyclic heterocyclyl group in which any two rings share two carbon atoms that are not directly connected to each other, wherein the bridged heterocyclyl may contain one or more double bonds. In the bridged heterocyclyl, one or more of the ring atoms are heteroatoms selected from the group consisting of nitrogen, oxygen, sulfur, S(O) and S(O)₂, and the remaining ring atoms are carbon atoms. The bridged heterocyclyl is preferably 6-14 (e.g., 6, 7, 8, 9, 10, 11, 12, 13 and 14) membered and more preferably 7-10 (e.g., 7, 8, 9 or 10) membered. According to the number of the formed rings, the bridged heterocyclyl may be bicyclic, tricyclic, tetracyclic or polycyclic, preferably bicyclic, tricyclic or tetracyclic, and more preferably bicyclic or tricyclic. Non-limiting examples of bridged heterocyclyl include:

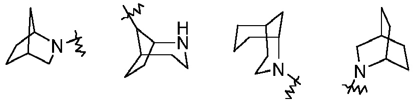

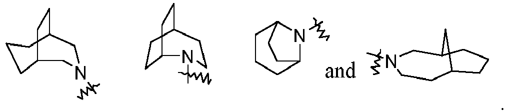

The heterocyclyl ring includes those in which the heterocyclyl described above (including monocyclic, spiro heterocyclic, fused heterocyclic and bridged heterocyclic rings) is fused to an aryl, heteroaryl or cycloalkyl ring, wherein the ring connected to the parent structure is heterocyclyl. Non-limiting examples include: 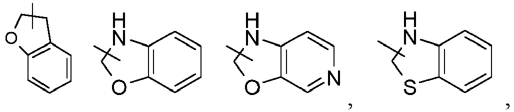 etc.

The heterocyclyl may be substituted or unsubstituted. When substituted, the substituent may be substituted at any available connection site, and the substituent is preferably one or more substituents independently optionally selected from the group consisting of a hydrogen atom, halogen, alkyl, alkoxy, haloalkyl, haloalkoxy, cycloalkyloxy, heterocyclyloxy, hydroxy, hydroxyalkyl, cyano, amino, nitro, cycloalkyl, heterocyclyl, aryl and heteroaryl.

The term "aryl" refers to a 6-14 membered, preferably 6-10 membered carbon monocyclic or fused polycyclic (fused polycyclic rings are those sharing a pair of adjacent carbon atoms) group having a conjugated π-electron system, such as phenyl and naphthyl. The aryl ring includes those in which the aryl ring described above is fused to a heteroaryl, heterocyclyl or cycloalkyl ring, wherein the ring connected to the parent structure is an aryl ring. Non-limiting examples include:

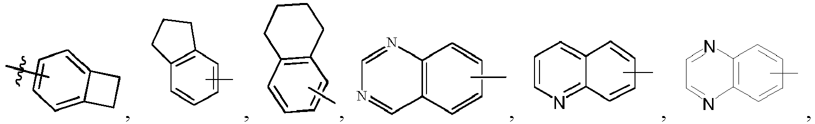

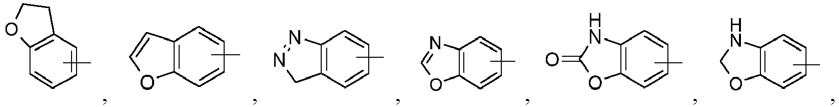

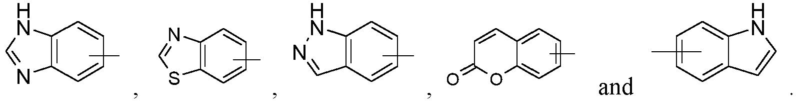

The aryl may be substituted or unsubstituted. When substituted, the substituent may be substituted at any available connection site, and the substituent is preferably one or more substituents independently optionally selected from the group consisting of a hydrogen atom, halogen, alkyl, alkoxy, haloalkyl, haloalkoxy, cycloalkyloxy, heterocyclyloxy, hydroxy, hydroxyalkyl, cyano, amino, nitro, cycloalkyl, heterocyclyl, aryl and heteroaryl. The term "heteroaryl" refers to a heteroaromatic system containing 1 to 4 (e.g., 1, 2, 3, and 4) heteroatoms and 5 to 14 ring atoms, wherein the heteroatoms are selected from the group consisting of oxygen, sulfur and nitrogen. The heteroaryl is preferably 5-10 membered (e.g., 5, 6, 7, 8, 9 or 10 membered) and more preferably 5- or 6-membered, e.g., furanyl, thienyl, pyridinyl, pyrrolyl, N-alkylpyrrolyl, pyrimidinyl, pyrazinyl, pyridazinyl, imidazolyl, pyrazolyl, triazolyl and tetrazolyl. The heteroaryl ring includes those in which the heteroaryl ring described above is fused to an aryl, heterocyclyl or cycloalkyl ring, wherein the ring connected to the parent structure is a heteroaryl ring. Non-limiting examples include:

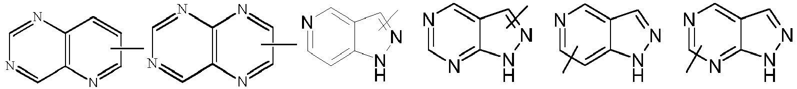

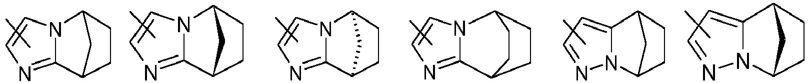

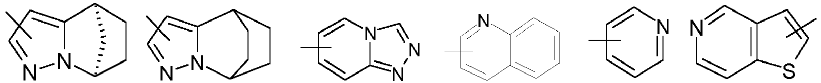

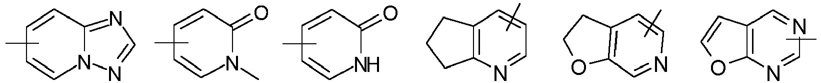

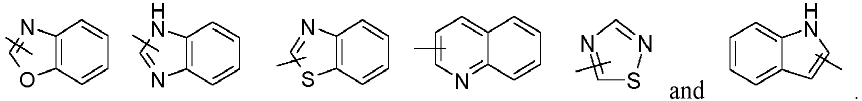

The heteroaryl may be substituted or unsubstituted. When substituted, the substituent may be substituted at any available connection site, and the substituent is preferably one or more substituents independently optionally selected from the group consisting of halogen, alkyl, alkoxy, haloalkyl, haloalkoxy, cycloalkyloxy, heterocyclyloxy, hydroxy, hydroxyalkyl, cyano, amino, nitro, cycloalkyl, heterocyclyl, aryl and heteroaryl.

The cycloalkyl, heterocyclyl, aryl and heteroaryl described above include residues derived from the parent ring by removal of one hydrogen atom from a ring atom, or residues derived from the parent ring by removal of two hydrogen atoms from the same ring atom or two different ring atoms, i.e., "divalent cycloalkyl", "divalent heterocyclyl", "arylene", or "heteroarylene".

The term "amino protecting group" refers to a group that can be easily removed and is intended to protect an amino group from being changed when a reaction is conducted elsewhere in the molecule. Non-limiting examples include (trimethylsilyl)ethoxymethyl, tetrahydropyranyl, *tert*-butoxycarbonyl, acetyl, benzyl, allyl, *p*-methoxybenzyl, and the like. These groups may be optionally substituted with 1 to 3 substituents selected from the group consisting of halogen, alkoxy and nitro. The amino protecting groups are preferably (trimethylsilyl)ethoxymethyl and *tert*-butoxycarbonyl.

The term "hydroxy protecting group" is a suitable group known in the art for protecting hydroxy. As an example, preferably, the hydroxy protecting group may be (C₁₋₁₀alkyl or aryl)₃silyl, e.g., triethylsilyl, triisopropylsilyl, *tert*-butyldimethylsilyl or *tert-*butyldiphenylsilyl; C₁₋₁₀ alkyl or substituted alkyl, preferably alkoxy or aryl-substituted alkyl, and more preferably C₁₋₆ alkoxy-substituted C₁₋₆ alkyl or phenyl-substituted C₁₋₆ alkyl, and most preferably C₁₋₄ alkoxy-substituted C₁₋₄ alkyl, e.g., methyl, *tert*-butyl, allyl, benzyl, methoxymethyl (MOM), ethoxyethyl or 2-tetrahydropyranyl (THP); (C₁₋₁₀ alkyl or aryl)acyl, e.g., formyl, acetyl, benzoyl or *p*-nitrobenzoyl; (C₁₋₆ alkyl or C₆₋₁₀ aryl)sulfonyl; or (C₁₋₆ alkoxy or C₆₋₁₀ aryloxy)carbonyl. The hydroxy protecting group is preferably *p*-nitrobenzoyl.

The term "cycloalkyloxy" refers to cycloalkyl-O-, wherein the cycloalkyl is defined as above.

The term "heterocyclyloxy" refers to the heterocyclyl-O-, wherein the heterocyclyl is defined as above.

The term "alkylthio" refers to alkyl-S-, wherein the alkyl is defined as above.

The term "haloalkyl" refers to alkyl substituted with one or more halogens, wherein the alkyl is defined as above.

The term "haloalkoxy" refers to alkoxy substituted with one or more halogens, wherein the alkoxy is defined as above.

The term "deuterated alkyl" refers to alkyl substituted with one or more deuterium atoms, wherein the alkyl is defined as above.

The term "hydroxyalkyl" refers to alkyl substituted with hydroxy, wherein the alkyl is defined as above.

The term "halogen" refers to fluorine, chlorine, bromine or iodine.

The term "hydroxy" refers to -OH.

The term "mercapto" refers to -SH.

The term "amino" refers to -NH₂.

The term "cyano" refers to -CN.

The term "nitro" refers to -NO₂.

The term "oxo" refers to "=O".

The term "carbonyl" refers to C=O.

The term "carboxyl" refers to -C(O)OH.

The term "carboxylate" refers to -C(O)O(alkyl), -C(O)O(cycloalkyl), (alkyl)C(O)O- or (cycloalkyl)C(O)O-, wherein the alkyl and cycloalkyl are defined as above.

The present disclosure also includes various deuterated forms of the compounds of general formulas (I), (IIaa), (IIaa-1), (IIaa-2), (Ilbb), (IIcc), (IIcc-1), (IIIaa), (IIIbb) and (IIIcc). Each available hydrogen atom connected to a carbon atom may be independently substituted with a deuterium atom. Those skilled in the art are able to synthesize the deuterated forms of the compounds of general formulas (I), (IIaa), (IIaa-1), (IIaa-2), (Ilbb), (IIcc), (IIcc-1), (IIIaa), (IIIbb) and (IIIcc) with reference to related literatures. Commercially available deuterated starting materials can be used in preparing the deuterated forms of the compound of formula (I), or they can be synthesized using conventional techniques with deuterated reagents including, but not limited to, deuterated borane, tri-deuterated borane in tetrahydrofuran, deuterated lithium aluminum hydride, deuterated iodoethane, deuterated iodomethane, and the like. Deuterides can generally retain comparable activity to non-deuterated compounds and can achieve better metabolic stability when deuterated at certain specific sites, thereby achieving certain therapeutic advantages.

The term "optional" or "optionally" means that the event or circumstance subsequently described may, but not necessarily, occur, and that the description includes instances where the event or circumstance occurs or does not occur. For example, "heterocyclyl optionally substituted with alkyl" means that alkyl may be, but not necessarily, present, and that the description includes instances where the heterocyclyl is or is not substituted with the alkyl.

The term "substituted" means that one or more, preferably 1-5, more preferably 1-3 hydrogen atoms in the group are independently substituted with a corresponding number of substituents. Those skilled in the art are able to determine (experimentally or theoretically) possible or impossible substitution without undue efforts. For example, it may be unstable when an amino or hydroxy group having a free hydrogen is bound to a carbon atom having an unsaturated (e.g., olefinic) bond.

The term "pharmaceutical composition" refers to a mixture containing one or more of the compounds described herein or a physiologically/pharmaceutically acceptable salt or prodrug thereof, and other chemical components, and other components, for example physiologically/pharmaceutically acceptable carriers and excipients. The purpose of the pharmaceutical composition is to promote the administration to an organism, which facilitates the absorption of the active ingredient, thereby exerting biological activities. The term "pharmaceutically acceptable salt" refers to salts of the disclosed compounds that are safe and effective for use in the body of a mammal and possess the requisite biological activities. The salts may be prepared separately during the final separation and purification of the compound, or by reacting an appropriate group with an appropriate base or acid. Bases commonly used to form pharmaceutically acceptable salts include inorganic bases such as sodium hydroxide and potassium hydroxide, and organic bases such as ammonia. Acids commonly used to form pharmaceutically acceptable salts include inorganic acids and organic acids.

For drugs and pharmacological active agents, the term "therapeutically effective amount" refers to an amount of a medicament or an agent that is sufficient to provide the desired effect but is non-toxic. The determination of the effective amount varies from person to person. It depends on the age and general condition of a subject, as well as the particular active substance used. The appropriate effective amount in a case may be determined by those skilled in the art in the light of routine tests.

The term "pharmaceutically acceptable" as used herein means that those compounds, materials, compositions and/or dosage forms which are, within the scope of reasonable medical judgment, suitable for use in contact with the tissues of patients without excessive toxicity, irritation, allergic response, or other problems or complications, and are commensurate with a reasonable benefit/risk ratio and effective for the intended use.

As used herein, the singular forms "a", "an" and "the" include plural references and vice versa, unless otherwise clearly defined in the context.

When the term "about" is applied to parameters such as pH, concentration and temperature, it means that the parameter may vary by ±10%, and sometimes more preferably within ±5%. As will be appreciated by those skilled in the art, when the parameters are not critical, the numbers are generally given for illustrative purposes only and are not intended to be limiting.

The compound disclosed herein may also include an isotopic derivative thereof. The term "isotopic derivative" refers to compounds that differ in structure only by having one or more enriched isotopic atoms. For example, compounds with the structure disclosed herein having "deuterium" or "tritium" in place of hydrogen, or ¹⁸F-fluorine labeling (¹⁸F isotope) in place of fluorine, or ¹¹C-, ¹³C- or ¹⁴C-enriched carbon (¹¹C-, ¹³C- or ¹⁴C-carbon labeling; ¹¹C-, ¹³C- or ¹⁴C-isotope) in place of a carbon atom are within the scope of the present disclosure. Such a compound can be used as an analytical tool or a probe in, for example, a biological assay, or may be used as a tracer for *in vivo* diagnostic imaging of disease, or as a tracer in a pharmacodynamic, pharmacokinetic or receptor study.

### Synthesis Method of Compounds of Present Disclosure

In order to achieve the purpose of the present disclosure, the following technical schemes are adopted in the present disclosure:

### Scheme 1

Provided is a method for preparing the compound of general formula (IV) or the tautomer, mesomer, racemate, enantiomer or diastereomer thereof or the mixture thereof, or the pharmaceutically acceptable salt thereof of the present disclosure, which comprises the following step: 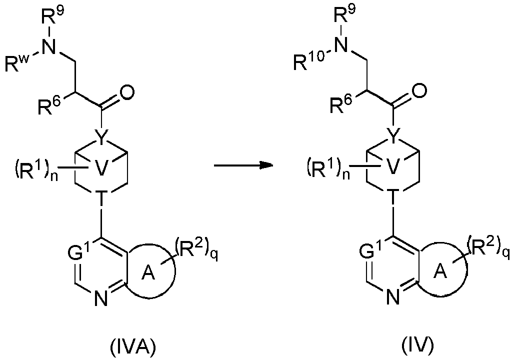 removing an amino protecting group from a compound of general formula (IVA) in an acidic condition to obtain the compound of formula (IV),
wherein:
R^{w} is the amino protecting group, preferably *tert*-butoxycarbonyl;
R¹⁰ is a hydrogen atom; and
R¹, R², R⁶, R⁹, Y, V, T, ring A, G¹, n and q are defined as in general formula (IV).

The reagents that provide acidic conditions in the above synthesis scheme include, but are not limited to, trifluoroacetic acid, hydrochloric acid, a solution of hydrogen chloride in 1,4-dioxane, formic acid, acetic acid, sulfuric acid, methanesulfonic acid, nitric acid, phosphoric acid, *p*-toluenesulfonic acid, Me₃SiCl an TMSOTf, preferably trifluoroacetic acid.

The above reactions are preferably conducted in a solvent including, but not limited to: ethylene glycol dimethyl ether, acetic acid, methanol, ethanol, *n*-butanol, toluene, tetrahydrofuran, dichloromethane, petroleum ether, ethyl acetate, *n*-hexane, dimethyl sulfoxide, 1,4-dioxane, water, *N*,*N-*dimethylformamide, and mixtures thereof.

### Scheme 2

Provided is a method for preparing the compound of general formula (IIcc-1) or the tautomer, mesomer, racemate, enantiomer or diastereomer thereof or the mixture thereof, or the pharmaceutically acceptable salt thereof of the present disclosure, which comprises: 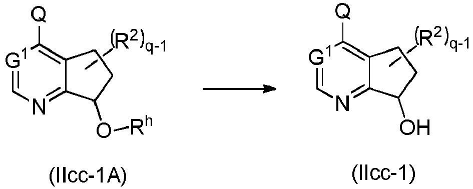 removing a hydroxy protecting group R^{h} from a compound of general formula (IIcc-1A) under an alkaline condition to obtain the compound of general formula (IIcc-1), wherein:
R^{h} is a hydroxy protecting group, preferably 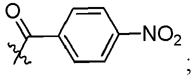
G¹, Q, R² and q are defined as in the compound of general formula (IIcc-1).

The reagents that provide alkaline conditions in the above synthesis scheme include organic bases including, but not limited to, triethylamine, *N,N-*diisopropylethylamine, *n-*butyllithium, lithium diisopropylamide, potassium acetate, sodium *tert*-butoxide and potassium *tert*-butoxide; and inorganic bases including, but not limited to, sodium hydride, potassium phosphate, sodium carbonate, sodium acetate, potassium acetate, potassium carbonate or cesium carbonate, sodium hydroxide, lithium hydroxide and potassium hydroxide; and are preferably lithium hydroxide.

The above reactions are preferably conducted in a solvent including, but not limited to: ethylene glycol dimethyl ether, acetic acid, methanol, ethanol, *n*-butanol, toluene, tetrahydrofuran, dichloromethane, petroleum ether, ethyl acetate, *n*-hexane, dimethyl sulfoxide, 1,4-dioxane, water, *N*,*N*-dimethylformamide, and mixtures thereof.

### DETAILED DESCRIPTION

The following examples further illustrate the present disclosure, but the present disclosure is not limited thereto.

### Examples

The structure of the compound was determined by nuclear magnetic resonance (NMR) spectroscopy and/or mass spectrometry (MS). NMR shift (δ) is given in a unit of 10⁻⁶ (ppm). NMR spectra were measured using a Bruker AVANCE-400 nuclear magnetic resonance instrument or Bruker AVANCE NEO 500M, with deuterated dimethyl sulfoxide (DMSO-*d₆*), deuterated chloroform (CDCl₃) and deuterated methanol (CD₃OD) as determination solvents and tetramethylsilane (TMS) as an internal standard.

Mass spectra were measured using Agilent 1200/1290 DAD-6110/6120 Quadrupole MS liquid chromatography-mass spectrometry system (manufacturer: Agilent; MS model: 6110/6120 Quadrupole MS), Waters ACQuity UPLC-QD/SQD (manufacturer: Waters, MS model: Waters ACQuity Qda Detector/Waters SQ Detector) and THERMO Ultimate 3000-Q Exactive (manufacturer: THERMO, MS model: THERMO Q Exactive).

High performance liquid chromatography (HPLC) was performed using the following HPLC instruments: Agilent HPLC 1200DAD, Agilent HPLC 1200VWD and Waters HPLC e2695-2489.

Chiral HPLC was performed on Agilent 1260 DAD HPLC.

HPLC preparation was performed using Waters 2545-2767, Waters 2767-SQ Detecor2, Shimadzu LC-20AP and Gilson GX-281 preparative chromatographs.

Chiral preparation was performed on a Shimadzu LC-20AP preparative chromatograph. A CombiFlash Rf200 (TELEDYNE ISCO) system was used for rapid preparation. Huanghai HSGF254 or Qingdao GF254 silica gel plates of specifications 0.15 mm to 0.2 mm were adopted for thin layer chromatography (TLC) analysis and 0.4 mm to 0.5 mm for TLC separation and purification.

The silica gel column chromatography generally used 200 to 300-mesh silica gel (Huanghai, Yantai) as the carrier.

The mean inhibition of kinase and the IC₅₀ value were measured using a NovoStar microplate reader (BMG, Germany).

Known starting materials described herein may be synthesized using or according to methods known in the art, or may be purchased from ABCR GmbH & Co. KG, Acros Organics, Aldrich Chemical Company, Accela ChemBio Inc., Chembee Chemicals, and other companies.

In the examples, the reactions can be performed in an argon atmosphere or a nitrogen atmosphere unless otherwise specified.

The argon atmosphere or nitrogen atmosphere means that the reaction flask is connected to a balloon containing about 1 L of argon or nitrogen.

A hydrogen atmosphere means that the reaction flask is connected to a balloon containing about 1 L of hydrogen.

Parr 3916EKX hydrogenator, Qinglan QL-500 hydrogenator or HC2-SS hydrogenator was used in the pressurized hydrogenation reactions.

The hydrogenation reactions usually involve 3 cycles of vacuumization and hydrogen purge.

A CEM Discover-S 908860 microwave reactor was used in the microwave reactions.

In the examples, a solution refers to an aqueous solution unless otherwise specified.

In the examples, the reaction temperature was room temperature, i.e., 20 °C to 30 °C, unless otherwise specified.

The monitoring of the reaction progress in the examples was conducted by thin layer chromatography (TLC). The developing solvent for reactions, the eluent system for column chromatography purification and the developing solvent system for thin layer chromatography included: A: dichloromethane/methanol system, B: *n*-hexane/ethyl acetate system, and C: petroleum ether/ethyl acetate system. The volume ratio of the solvents was adjusted according to the polarity of the compound, or by adding a small amount of basic or acidic reagents such as triethylamine and acetic acid.

The Boc is *tert*-butyloxycarbonyl.

### Example 1

### (S)-1-((1R,5S)-3-(7H-pyrrolo[2,3-d]pyrimidin-4-yl)-3,8-diazabicyclo[3.2.1]octan-8-yl)-2-(4-chlorophenyl)-3-(isopropylamino)propan-1-one 1

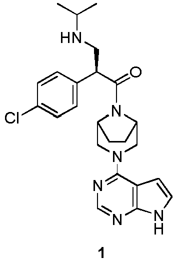

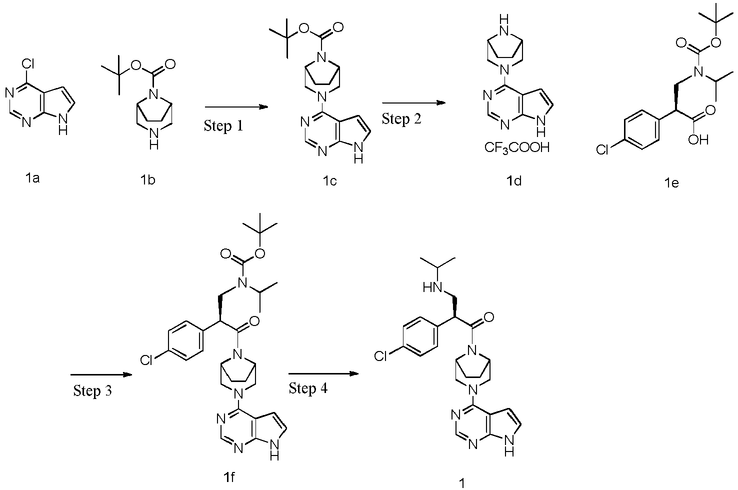

### Step 1

### Tert-butyl (1R,5S)-3-(7H-pyrrolo[2,3-d]pyrimidin-4-yl)-3,8-diazabicyclo[3.2.1]octane-8-carboxylate 1c

4-chloropyrrolopyrimidine **1a** (75 mg, 0.48 mmol, Bide Pharmatech Ltd.), *tert*-butyl (1*R*,5*S*)-3,8-diazabicyclo[3.2.1]octane-8-carboxylate **1b** (100 mg, 0.48 mmol, Bide Pharmatech Ltd) and *N,N-*diisopropylethylamine (182 mg, 1.41 mmol) were dissolved in 3 mL of ethanol, and the mixture was heated to 80 °C and stirred for 14 h. The reaction solution was cooled to room temperature and concentrated under reduced pressure, and the residue was purified by column chromatography with a developing solvent system A to obtain the title compound 1c (110 mg, yield: 70.8%).

MS m/z (ESI): 330.4 [M+1].

### Step 2

### 4-((1R,5S)-3,8-diazabicyclo[3.2.1]octan-3-yl)-7H-pyrrolo[2,3-d]pyrimidine trifluoroacetate 1d

Compound **1c** (38 mg, 0.09 mmol) was dissolved in 3 mL of dichloromethane solvent, and 0.5 mL of trifluoroacetic acid was added. The mixture was stirred for 2 h. The reaction solution was concentrated under reduced pressure to obtain the title compound **1d** (crude product, 30 mg).

MS m/z (ESI): 230.2 [M+1].

### Step 3

*Tert*-butyl ((*S*)-(3-((1*R*,5*S*)-3-(7*H*-pyrrolo[2,3-*d*]pyrimidin-4-yl)-3,8-diazabicyclo[3.2.1]octan-8-yl)-2-(4-chlorophenyl)-3-oxopropyl)(isopropyl)carbamate **1f** Compound **1d** (51 mg, 100 µmol) and (*S*)-3-((*tert*-butyloxycarbonyl)(isopropyl)amino)-2-(4-chlorophenyl)propanoic acid **1e** (50 mg, 100 µmol, prepared by the method disclosed in "J. Med. Chem. 2012, 55, 8110-8127") were dissolved in 2 mL of *N,N-*dimethylformamide, and then 2-(7-benzotriazole oxide)-*N,N,N',N'*-tetramethyluronium hexafluorophosphate (36 mg, 0.15 mmol) and *N,N*-diisopropylethylamine (87 mg, 0.67 mmol) were added. The mixture was stirred for 2 h. The reaction solution was diluted with ethyl acetate (20 mL), washed with water, and concentrated under reduced pressure to obtain the title compound **1f** (crude product, 35 mg), which was used directly in the next step.

MS m/z (ESI): 553.1 [M+1].

### Step 4

### (S)-1-((1R,5S)-3-(7H-pyrrolo[2,3-d]pyrimidin-4-yl)-3,8-diazabicyclo[3.2.1]octan-8-yl)-2-(4-chlorophenyl)-3-(isopropylamino)propan-1-one 1

Compound **1f** (crude product, 35 mg, 63 µmol) was dissolved in 3 mL of dichloromethane solvent, and then 0.5 mL of trifluoroacetic acid was added. The mixture was stirred for 2 h. The reaction solution was concentrated under reduced pressure, and the residue was purified by preparative chromatography to obtain the title compound **1** (15 mg, yield: 52.3%).

MS m/z (ESI): 453.2 [M+1].

¹H NMR (400 MHz, CD₃OD): δ 8.13 (d, 1H), 7.40-7.43 (m, 4H), 7.14 (d, 1H), 6.64 (d, 1H), 4080-4.83 (m, 1H), 4.53-4.56 (m, 2H), 4.18-4.21 (m, 2H), 3.50-3.52 (m, 1H), 2.88 (d, 1H), 2.76-2.80 (m, 2H), 2.14-2.18 (m, 1H), 1.82-1.85 (m, 4H), 1.05-1.08 (m, 6H).

### Example 2

### 4-((1R,5S)-8-(S)-2-(4-chlorophenyl)-3-(isopropylamino)propanoyl)-3,8-diazabicyclo[3.2.1]octan-3-yl)-5,5-dimethyl-5,7-dihydro-6H-pyrrolo[2,3-d]pyrimidin-6-one 2

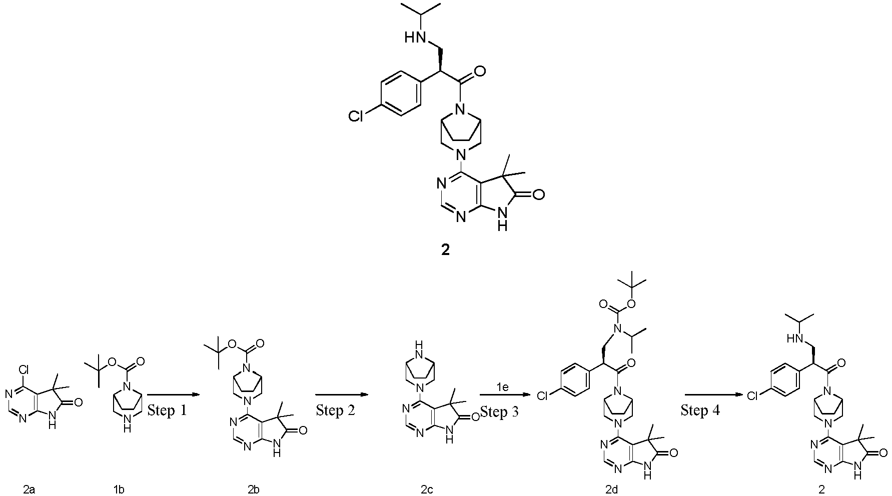

### Step 1

### Tert-butyl (1R,5S)-3-(5,5-dimethyl-6-oxo-6,7-dihydro-5H-pyrrolo[2,3-d]pyrimidin-4-yl)-3,8-diazabicyclo[3.2.1]octane-8-carboxylate 2b

Compound **1b** (250 mg, 1.18 mmol), 4-chloro-S,S-dimethyl-7 H-pyrrolo[2,3-*d*]pyrimidin-6-one **2a** (233 mg, 1.18 mmol, Bide Pharmatech Ltd.) and *N,N*-diisopropylethylamine (457 mg, 3.53 mmol) were dissolved in *N,N*-dimethylformamide (5 mL), and the mixture was stirred at 120 °C overnight. The reaction solution was cooled to room temperature and concentrated under reduced pressure, and the residue was purified by column chromatography with a developing solvent system C to obtain the title compound **2b** (220 mg, yield: 50.0%).

MS m/z (ESI): 374.1 [M+1].

### Step 2

### 4-((1R,5S)-3,8-diazabicyclo[3.2.1]octan-3-yl)-5,5-dimethyl-5,7-dihydro-6H-pyrrolo[2,3-d]pyrimidin-6-one 2c

Compound **2b** was dissolved in a solution of hydrogen chloride in dioxane (4 M, 5 mL) and the mixture was stirred at room temperature for 1 h. The reaction solution was concentrated under reduced pressure to obtain the title compound **2c** (crude product), which was used directly in the next step.

MS m/z (ESI): 274.1 [M+1].

### Step 3

### Tert-butyl ((S)-2-(4-chlorophenyl)-3-((1R,5S)-3-(5,5-dimethyl-6-oxo-6,7-dihydro-5H-pyrrolo[2,3-d]pyrimidin-4-yl)-3,8-diazabicyclo[3.2.1]octan-8-yl)-3-oxopropyl)(isopropyl)carbamate 2d

Compound **2c** (160 mg, 585 µmol) and compound **1e** (200 mg, 585 µmol) were dissolved in 5 mL of dichloromethane, and 2-(7-benzotriazole oxide)-*N,N,N',N'-*tetramethyluronium hexafluorophosphate (334 mg, 878 µmol) and triethylamine (237 mg, 2.34 mmol) were added. The mixture was stirred overnight. The reaction solution was diluted with ethyl acetate (20 mL), washed with water, and concentrated under reduced pressure to obtain the title compound **2d** (crude product, 130 mg), which was used directly in the next step.

MS m/z (ESI): 597.1 [M+1].

### Step 4

### 4-((1R,5S)-8-(S)-2-(4-chlorophenyl)-3-(isopropylamino)propanoyl)-3,8-diazabicyclo[3.2.1]octan-3-yl)-5,5-dimethyl-5,7-dihydro-6H-pyrrolo[2,3-d]pyrimidin-6-one 2

Compound **2d** (crude product) was dissolved in ethyl acetate (2 mL), and a solution of hydrogen chloride in dioxane (4 M, 5 mL) was added dropwise with stirring at room temperature. The mixture was stirred at room temperature for 1 h. The reaction solution was concentrated under reduced pressure, and the residue was purified by preparative chromatography to obtain the title compound **2** (30 mg, yield: 27.7%).

MS m/z (ESI): 497.2 [M+1].

¹H NMR (600 MHz, CD₃OD): δ 8.21 (d, 1H), 7.41-7.35 (m, 4H), 4.83-4.82 (m, 1H), 4.56-4.43 (m, 2H), 4.34-4.27 (m, 2H), 4.17-4.09 (m, 1H), 3.90-3.87 (m, 1H), 2.99-2.97 (m, 1H), 2.91-2.87 (m, 1H), 2.82-2.77 (m, 1H), 2.11-2.07 (m, 1H), 2.01-1.99 (m, 1H), 1.96-1.90 (m, 1H), 1.86-1.80 (m, 1H), 1.74-1.66 (m, 2H), 1.51-1.50 (m, 2H), 1.41 (s, 2H), 1.32 (s, 2H), 1.13-1.09 (m, 6H).

### Example 3

### (S)-2-(4-chlorophenyl)-3-(isopropylamino)-1-((1R,5S)-3-(5-methyl-7H-pyrrolo[2,3-d]pyrimidin-4-yl)-3,8-diazabicyclo[3.2.1]octan-8-yl]propan-1-one 3

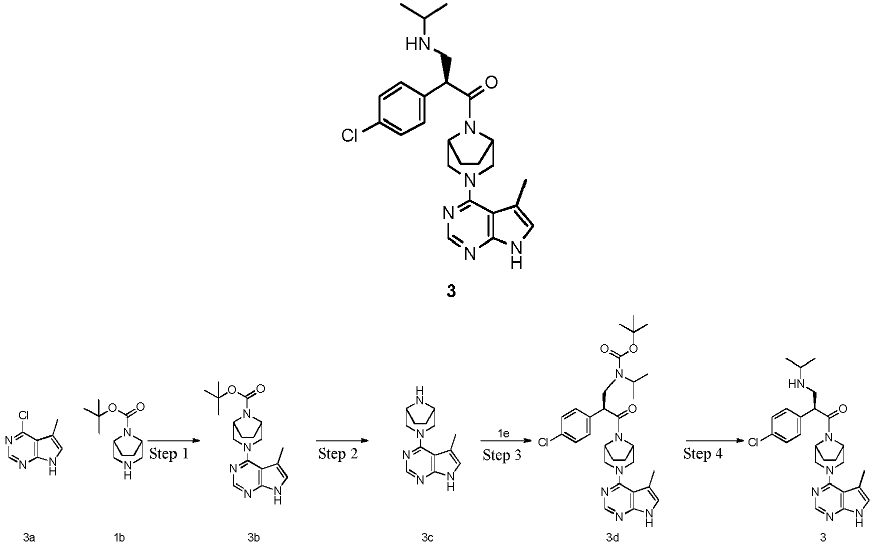

### Step 1

### Tert-butyl (1R,5S)-3-(5-methyl-7H-pyrrolo[2,3-d]pyrimidin-4-yl)-3,8-diazabicyclo[3.2.1]octane-8-carboxylate 3b

Compound **1b** (254 mg, 1.20 mmol), 4-chloro-5-methyl-7*H*-pyrrolo[2,3-*d*] pyrimidine **3a** (200 mg, 1.19 mmol, WuXi AppTec Co. Ltd.) and *N*,*N*-diisopropylethylamine (462 mg, 3.57 mmol) were dissolved in *N*,*N*-dimethylformamide (6 mL), and the mixture was stirred at 80 °C overnight. The reaction solution was cooled to room temperature and concentrated under reduced pressure, and the residue was purified by column chromatography with a developing solvent system A to obtain the title compound **3b** (220 mg, yield: 61%).

MS m/z (ESI): 344.2 [M+1].

### Step 2

4-((1*R*,5*S*)-3,8-diazabicyclo[3.2.1]octan-3-yl)-5-methyl-7*H*-pyrrolo[2,3-*d*]pyrimidine **3c** Compound **3b** (250 mg, 728 µmol) was dissolved in dichloromethane (5 mL), trifluoroacetic acid (415 mg, 3.64 mmol) was added, and the mixture was stirred at room temperature for 1 h. The reaction solution was concentrated under reduced pressure to obtain the title compound **3c** (crude product, 177 mg), which was used directly in the next step.

MS m/z (ESI): 244.1 [M+1].

### Step 3

### Tert-butyl ((S)-2-(4-chlorophenyl)-3-((1R,5S)-3-(5-methyl-7H-pyrrolo[2,3-d]pyrimidin-4-yl)-3,8-diazabicyclo[3.2.1]octan-8-yl)-3-oxopropyl)(isopropyl)carbamate 3d

Compound **3c** (177 mg, 727 µmol) and compound **1e** (331 mg, 726 µmol) were dissolved in 5 mL of *N*,*N*-dimethylformamide, and 2-(7-benzotriazole oxide)-*N*,*N*,*N*,*N-*tetramethyluronium hexafluorophosphate (256 mg, 1.09 mmol) and *N*,*N-*diisopropylethylamine (282 mg, 2.18 mmol) were added. The mixture was reacted at room temperature for 2 h. The reaction solution was diluted with ethyl acetate (20 mL), washed with water, and concentrated under reduced pressure to obtain the title compound **3d** (crude product, 200 mg), which was used directly in the next step.

MS m/z (ESI): 567.1 [M+1].

### Step 4

### (S)-2-(4-chlorophenyl)-3-(isopropylamino)-1-((1R,5S)-3-(5-methyl-7H-pyrrolo[2,3-d]pyrimidin-4-yl)-3,8-diazabicyclo[3.2.1]octan-8-yl]propan-1-one 3

Compound **3d** (crude product, 200 mg, 353 µmol) was dissolved in dichloromethane (5 mL), trifluoroacetic acid (201 mg, 1.76 mmol) was added, and the mixture was stirred at room temperature for 1 h. The reaction solution was concentrated under reduced pressure, and the residue was purified by preparative chromatography to obtain the title compound **3** (5 mg, yield: 3.0%).

MS m/z (ESI): 467.1 [M+1].

¹H NMR (600 MHz, CD₃OD): δ 8.16-8.09 (d, 1H), 7.48-7.27 (m, 4H), 6.99-6.93 (d, 1H), 4.83-4.79 (m, 1H), 4.42-4.25 (m, 3H), 3.78-3.75 (m, 1H), 3.65-3.57 (m, 1H), 3.51-3.43 (m, 1H), 3.37-3.32 (m, 1H), 3.19-3.09 (m, 2H), 2.17 (s, 3H), 2.04-1.66 (m, 4H), 1.37-1.27 (m, 6H).

### Example 4

### (S)-2-(4-chlorophenyl)-1-((1R,5S)-3-((5R,7R)-7-hydroxy-5-methyl-6,7-dihydro-5H-cyclopenta[d]pyrimidin-4-yl)-3,8-diazabicyclo[3.2.1]octan-8-yl)-3-(isopropylamino)propan-1-one 4

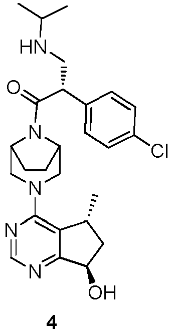

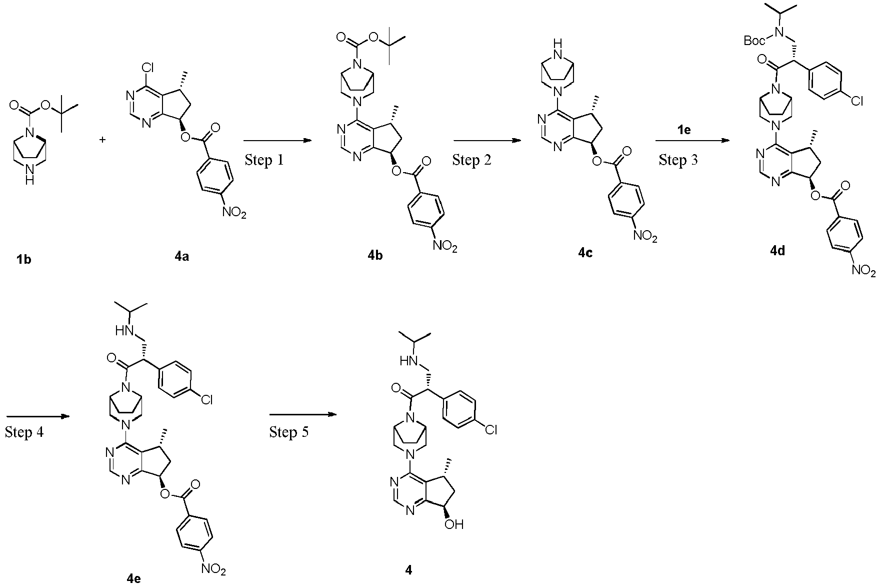

### Step 1

### Tert-butyl (1R,5S)-3-((5R,7R)-5-methyl-7-((4-nitrobenzoyl)oxy)-6,7-dihydro-5H-cyclopenta[d]pyrimidin-4-yl)-3,8-diazabicyclo[3.2.1]octane-8-carboxylate 4b

Compound **1b** (130 mg, 0.61 mmol, WuXi AppTec Co. Ltd.) was dissolved in isopropanol (10 mL), and **4a** (204 mg, 0.61 mmol, prepared according to the method disclosed in page 51 of the specification of patent application "CN104876921A") and diisopropylethylamine (227 mg, 2.14 mmol) were added at room temperature. The mixture was reacted at 80 °C for 24 h. The reaction solution was cooled to room temperature and concentrated under reduced pressure, and the residue was purified by column chromatography with an eluent system C to obtain the title compound **4b** (152 mg, yield: 49%).

MS m/z (ESI): 510.2 [M+1]⁺.

### Step 2

### (5R,7R)-4-((1R,5S)-3,8-diazabicyclo[3.2.1]octan-3-yl)-5-methyl-6,7-dihydro-5H-cyclopenta[d]pyrimidin-7-yl 4-nitrobenzoate 4c

Compound **4b** (190 mg, 0.37 mmol) was dissolved in a solution of hydrochloric acid in dioxane (4 M, 5 mL), and the mixture was reacted at room temperature for 1 h. The reaction solution was concentrated under reduced pressure to obtain the title compound **4c** (152 mg), which was used directly in the next step without purification.

MS m/z (ESI): 410.1 [M+1]⁺.

### Step 3

### (5R,7R)-4-((1R,5S)-8-((S)-3-(tert-butoxycarbonyl)(isopropyl)amino)-2-(4-chlorophenyl)propanoyl)-3,8-diazabicyclo[3.2.1]octan-3-yl)-5-methyl-6,7-dihydro-5H-cyclopenta[d]pyrimidin-7-yl 4-nitrobenzoate 4d

Compound **4c** (152 mg, 0.37 mmol) was dissolved in N,N-dimethylformamide (5 mL), and **1e** (127 mg, 0.37 mmol), 2-(7-azabenzotriazol)-tetramethyluronium hexafluorophosphate (183 mg, 0.48 mmol) and diisopropylethylamine (168 mg, 1.30 mmol) were added. The mixture was reacted at room temperature for 2 h. The reaction solution was diluted with ethyl acetate and washed with saturated sodium chloride solution. The organic phase was dried and concentrated under reduced pressure, and the residue was purified by column chromatography with an eluent system C to obtain the title compound **4d** (270 mg, yield: 99%).

MS m/z (ESI): 733.1 [M+1]⁺.

### Step 4

### (5R,7R)-4-((1R,5S)-8-((S)-2-(4-chlorophenyl)-3-(isopropylamino)propanoyl)-3,8-diazabicyclo[3.2.1]octan-3-yl)-5-methyl-6,7-dihydro-5H-cyclopenta[d]pyrimidin-7-yl 4-nitrobenzoate 4e

Compound **4d** (270 mg, 0.37 mmol) was dissolved in a solution of hydrochloric acid in dioxane (4 M, 5 mL), and the mixture was reacted at room temperature for 1 h. The reaction solution was concentrated under reduced pressure to obtain the title compound **4e** (230 mg), which was used directly in the next step without purification.

MS m/z (ESI): 633.1 [M+1]⁺.

### Step 5

### (S)-2-(4-chlorophenyl)-1-((1R,5S)-3-((5R,7R)-7-hydroxy-5-methyl-6,7-dihydro-5H-cyclopenta[d]pyrimidin-4-yl)-3,8-diazabicyclo[3.2.1]octan-8-yl)-3-(isopropylamino)propan-1-one 4

Compound **4e** (230 mg, 0.36 mmol) was dissolved in a mixed solvent of tetrahydrofuran (8 mL) and water (8 mL), lithium hydroxide (43.3 mg, 1.81 mmol) was added, and the mixture was stirred at room temperature for 1 h. The reaction solution was diluted with water and extracted three times with ethyl acetate, and the organic phases were combined, washed with saturated sodium chloride solution, dried and concentrated under reduced pressure. The residue was purified by liquid chromatography (instrument model: Gilson 281; chromatographic column: X-Bridge, Prep 30*150 mm; 5 µm, C18; mobile phase: A-water (10 mM ammonium bicarbonate) B-acetonitrile, flow rate: 30 mL/min; column temperature: room temperature) to obtain the title compound **4** (150 mg, yield: 85%).

MS m/z (ESI): 484.2 [M+1]⁺.

¹H NMR (500 MHz, CDCl₃): δ 8.41-8.55 (m, 1H), 7.25-7.38 (m, 4H), 5.06-5.16 (m, 1H), 4.80-4.88 (m, 1H), 4.10-4.51 (m, 3H), 3.76-3.97 (m, 2H), 3.12-3.49 (m, 3H), 2.71-2.96 (m, 3H), 1.98-2.27 (m, 3H), 1.53-1.90 (m, 4H), 1.00-1.21 (m, 9H).

### Example 5

### (2S)-1-(3-(7H-pyrrolo[2,3-d]pyrimidin-4-yl)-3,9-diazabicyclo[3.3.1]nonan-9-yl)-2-(4-chlorophenyl)-3-(isopropylamino)propan-1-one 5

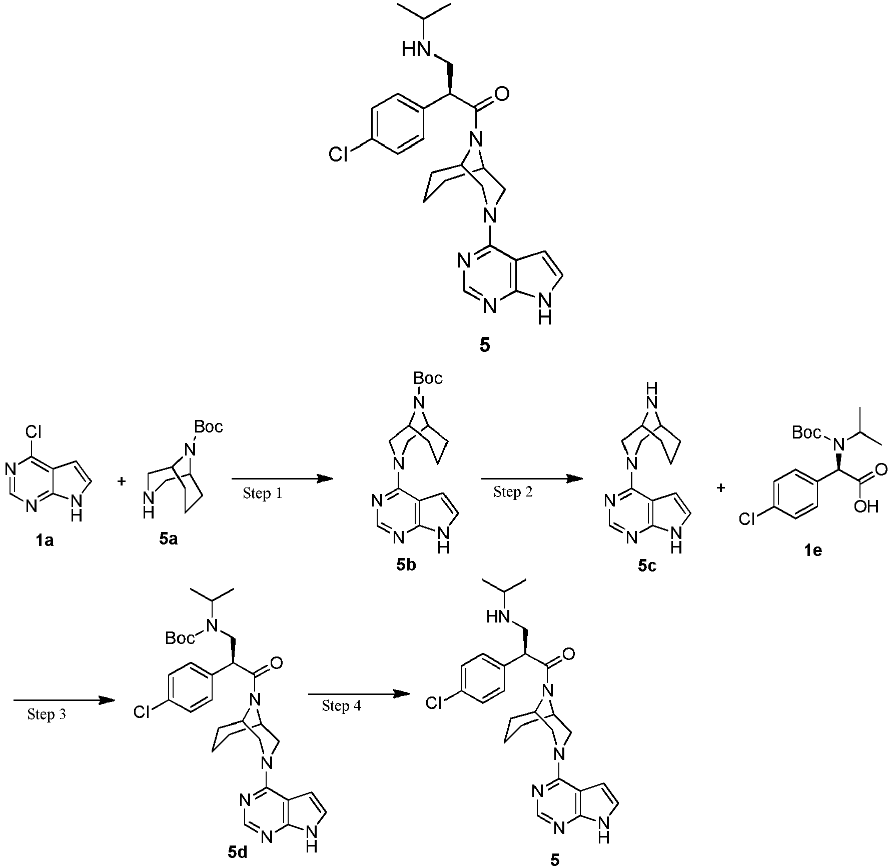

### Step 1

### Tert-butyl 3-(7H-pyrrolo[2,3-d]pyrimidin-4-yl)-3,9-diazabicyclo[3.3.1]nonane-9-carboxylate 5b

*Tert*-butyl 3,9-diazabicyclo[3.3.1]nonane-9-carboxylate **5a** (100 mg, 0.44 mmol, WuXi AppTec Co. Ltd.) was dissolved in ethanol (2.6 mL), and *N*,*N*-diisopropylethylamine (143 mg, 1.1 mmol) and 4-chloropyrrolopyrimidine **1a** (81.5 mg, 0.53 mmol) were added. The reaction mixture was reacted at 80 °C in a sealed tube overnight. The reaction mixture was cooled to room temperature, diluted with dichloromethane, transferred to a roundbottomed flask and concentrated to dryness by rotary evaporation, and the residue was dried with an oil pump to obtain the title compound **5b** (151.7 mg, 0.44 mmol), which was directly used in the next step.

MS m/z (ESI): 344.1 [M+1]⁺.

### Step 2

### 4-(3,9-diazabicyclo[3.3.1]nonan-3-yl)-7H-pyrrolo[2,3-d]pyrimidine 5c

Compound **5b** (151.7 mg, 0.44 mmol) was dissolved in dichloromethane (1.5 mL), and a solution of hydrogen chloride in 1,4-dioxane (4 M, 0.9 mL) was added. The mixture was reacted at room temperature for 2.5 h. The reaction solution was concentrated to dryness by rotary evaporation, and the residue was dried with an oil pump to obtain the title compound **5c** (102 mg, 0.42 mmol), which was directly used in the next step.

MS m/z (ESI): 244.1 [M+1]⁺.

### Step 3

### Tert-butyl ((2S)-3-(3-(7H-pyrrolo[2,3-d]pyrimidin-4-yl)-3,9-diazabicyclo[3.3.1]nonan-9-yl)-2-(4-chlorophenyl)-3-oxopropyl)(isopropyl)carbamate 5d

Compound **1e** (110 mg, 0.32 mmol) was dissolved in *N*,*N*-dimethylformamide (3 mL), and *N*,*N*-diisopropylethylamine (145.7 mg, 1.13 mmol), compound **5c** (102 mg, 0.42 mmol) and O-(7-azabenzotriazol-1-yl)-*N*,*N*,*N*',*N*'-tetramethyluronium hexafluorophosphate (183.7 mg, 0.48 mmol) were added. The reaction mixture was stirred at room temperature overnight. The reaction mixture was added with saturated aqueous ammonium chloride solution, extracted with ethyl acetate, washed with saturated sodium chloride solution, dried over anhydrous sodium sulfate, filtered and concentrated to dryness by rotary evaporation, and the residue was dried with an oil pump to obtain the title compound **5d** (82 mg, 0.14 mmol), which was used directly in the next step.

MS m/z (ESI): 567.3 [M+1]⁺.

### Step 4

### (2S)-1-(3-(7H-pyrrolo[2,3-d]pyrimidin-4-yl)-3,9-diazabicyclo[3.3.1]nonan-9-yl)-2-(4-chlorophenyl)-3-(isopropylamino)propan-1-one 5

Compound **5d** (82 mg, 0.14 mmol) was dissolved in dichloromethane (1 mL), and a solution of hydrogen chloride in 1,4-dioxane (4 M, 0.3 mL) was added. The mixture was reacted at room temperature for 2.5 h. The reaction solution was concentrated to dryness by rotary evaporation, and the residue was dried with an oil pump, dissolved in small amount of methanol and then purified by preparative chromatography (instrument model: Gilson 281; chromatographic column: X-Bridge, Prep 30*150 mm; 5 µm, C18; mobile phase: A-water (10 mM ammonium bicarbonate) B-acetonitrile, flow rate: 30 mL/min; column temperature: room temperature) to obtain the title compound **5** (8 mg, yield: 12%).

MS m/z (ESI): 467.2 [M+1]⁺.

¹H NMR (500 MHz, CD₃OD): *δ* 8.14 (d, 1H), 7.38-7.47 (m, 4H), 7.14 (dd, 1H), 6.64 (dd, 1H), 4.47-4.92 (m, 2H), 4.18-4.34 (m, 2H), 3.43-3.57 (m, 3H), 2.95-3.34 (m, 2H), 1.89-2.37 (m, 4H), 1.52-1.55 (m, 1H), 1.28-1.34 (m, 2H), 1.22 (d, 6H).

### Example 6

### (2S)-1-(7-(7H-pyrrolo[2,3-d]pyrimidin-4-yl)-3-oxa-7,9-diazabicyclo[3.3.1]nonan-9-yl)-2-(4-chlorophenyl)-3-(isopropylamino)propan-1-one 6

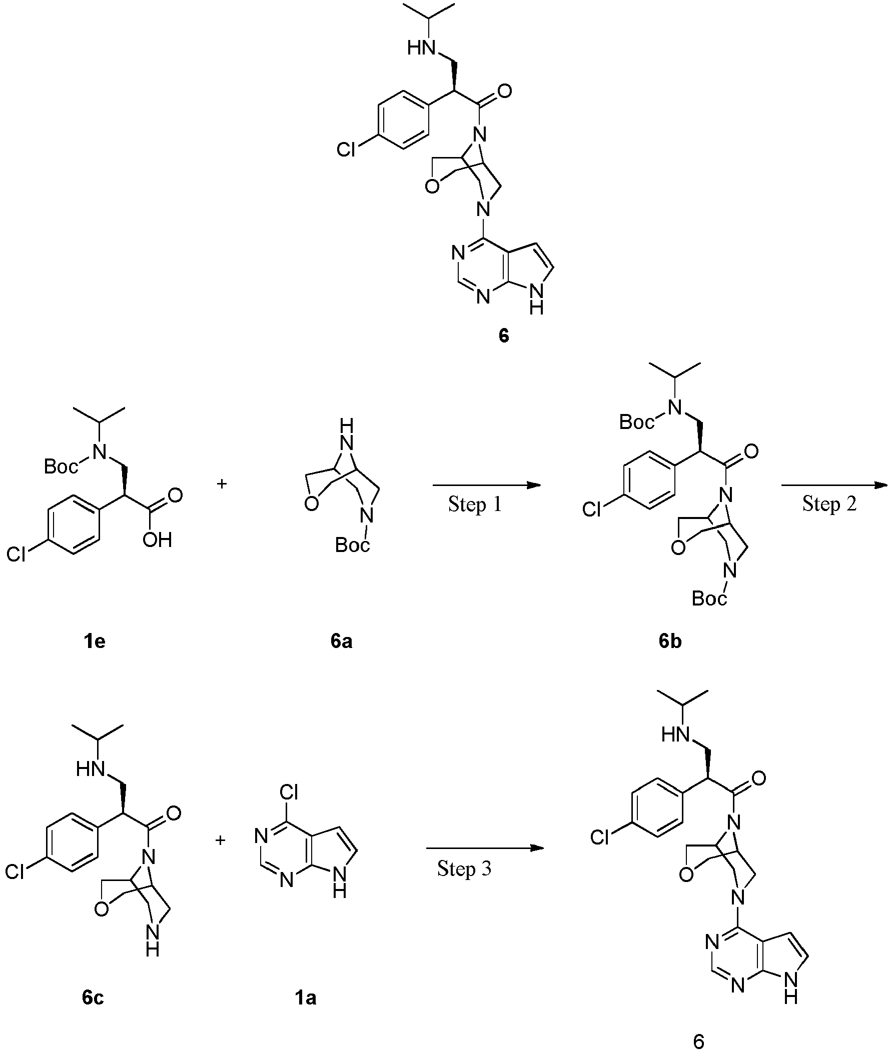

### Step 1

### Tert-butyl 9-((S)-3-(isopropylamino)-2-(4-chlorophenyl)propanoyl)-3-oxa-7,9-diazabicyclo[3.3.1]nonane-7-carboxylate 6b

*Tert*-butyl 3-oxa-7,9-diazabicyclo[3.3.1]nonane-9-carboxylate**6a** (67 mg, 293.49 µmol, PharmaBlock Sciences (Nanjing), Inc. ) and (*S*)-3-((*tert-*butyloxycarbonyl)(isopropyl)amino)-2-(4-chlorophenyl)propanoic acid **1e** (100 mg, 292.54 µmol, prepared by the method disclosed in "J. Med. Chem. 2012, 55, 8110-8127") were dissolved in 2 mL of *N*,*N*-dimethylformamide, and then 2-(7-benzotriazole oxide)-*N*,*N*,*N*',*N*'-tetramethyluronium hexafluorophosphate (166 mg, 436.58 µmol) and triethylamine (88 mg, 869.65 µmol) were added. The mixture was stirred for 2 h. The reaction solution was diluted with ethyl acetate (20 mL), washed with water, and concentrated under reduced pressure to obtain the title compound **6b** (crude product, 160 mg), which was used directly in the next step.

MS m/z (ESI): 552.9 [M+1].

### Step 2

### (2S)-1-(3-oxa-7,9-diazabicyclo[3.3.1]nonan-9-yl)-2-(4-chlorophenyl)-3-(isopropylamino)propan-1-one 6c

Compound **6b** (160 mg, 289.80 µmol) was dissolved in 3 mL of ethyl acetate solvent, and 2 mL of a solution of hydrochloric acid in dioxane (4 mol/L) was added. The mixture was stirred for 2 h. The reaction solution was concentrated under reduced pressure to obtain the title compound **6c** (crude product, 100 mg).

MS m/z (ESI): 352.9.[M+1].

### Step 3

### (2S)-1-(7-(7H-pyrrolo[2,3-d]pyrimidin-4-yl)-3-oxa-7,9-diazabicyclo[3.3.1]nonan-9-yl)-2-(4-chlorophenyl)-3-(isopropylamino)propan-1-one 6

4-chloropyrrolopyrimidine **1a** (50 mg, 325.58 mmol, Bide Pharmatech Ltd.), compound **6c** (100 mg, 287.03 mmol) and *N,N*-diisopropylethylamine (111 mg, 858.84 µmol) were dissolved in 3 mL of ethanol, and the mixture was heated to 80 °C and stirred for 14 h. The reaction solution was cooled to room temperature and concentrated under reduced pressure, and the residue was purified by preparative chromatography (instrument model: Gilson 281; chromatographic column: X-Bridge, Prep 30*150 mm; 5 µm, C18; mobile phase: A-water (10 mM ammonium bicarbonate) B-acetonitrile, flow rate: 30 mL/min; column temperature: room temperature) to obtain the title compound **6** (45 mg, yield: 33.4%).

MS m/z (ESI): 469.2 [M+1].

¹HNMR (500 MHz, CD₃OD): δ 8.14-8.09 (d, 1H), 7.45-7.36 (m, 4H), 7.15-7.10 (d, 1H), 6.73-6.57 (dd, 1H), 5.00-4.99 (m, 1H), 4.72-4.52 (m, 2H), 4.21-4.14 (m, 2H), 4.03-3.50 (m, 6H), 2.85-2.75 (m, 1H), 2.47-2.44 (m, 1H), 1.35-1.12 (m, 7H).

### Example 7

### (2S)-1-(9-(7H-pyrrolo[2,3-d]pyrimidin-4-yl)-3-oxa-7,9-diazabicyclo[3.3.1]nonan-7-yl)-2-(4-chlorophenyl)-3-(isopropylamino)-propan-1-one 7

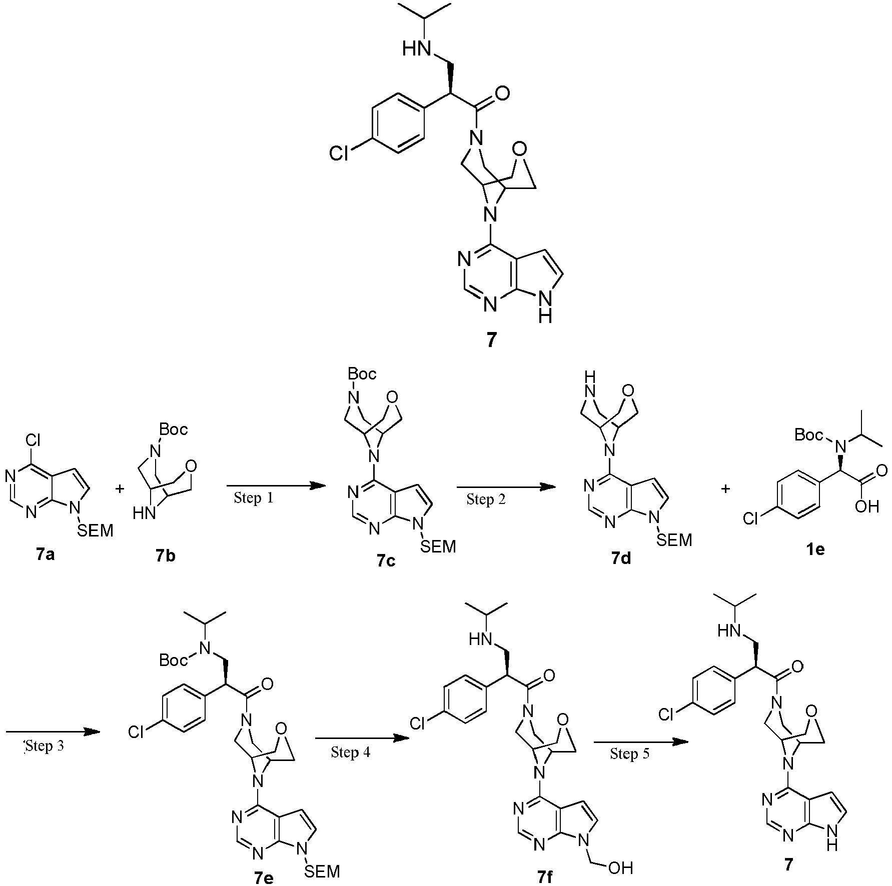

### Step 1

### Tert-butyl 9-(7-(((2-(trimethylsilyl)ethoxy)methyl)-7H-pyrrolo[2,3-d]pyrimidin-4-yl)-3-oxa-7,9-diazabicyclo[3.3.1]nonane-7-carboxylate 7c

To a 15 mL sealed tube were added 4-chloro-7-(((2-(trimethylsilyl)ethoxy)methyl)-7*H-*pyrrolo[2,3-*d*]pyrimidine **7a** (179 mg, 0.63 mmol, AK Limited), *tert*-butyl 3-oxa-7,9-diazabicyclo[3.3.1]nonane-7-carboxylate **7b** (125 mg, 0.55 mmol, WuXi AppTec Co. Ltd.), sodium *tert*-butoxide (158 mg, 1.64 mmol), tris(dibenzylideneacetone)dipalladium(0) (75.3 mg, 0.082 mmol, Frontier Scientific, Inc.) and 2-dicyclohexylphosphine-2'-(*N*,*N*-dimethylamine)-biphenyl (65 mg, 0.16 mmol, Bide Pharmatech Ltd.) successively, and then 1,4-dioxane (3.5 mL) was added. The reaction mixture was immediately purged with argon 3 times and reacted overnight at 110 °C under argon atmosphere. The reaction mixture was cooled to room temperature, diluted with ethyl acetate and filtered through celite, and the filter residue was washed with ethyl acetate, concentrated to dryness by rotary evaporation and purified by column chromatography with an eluent system (PE/EA = 1.5:1) to obtain the title compound **7c** (57 mg, 22%).

MS m/z (ESI): 476.2 [M+1]⁺.

### Step 2

### 9-(7-(((2-(trimethylsilyl)ethoxy)methyl)-7H-pyrrolo[2,3-d]pyrimidin-4-yl)-3-oxa-7,9-diazabicyclo[3.3.1]nonane 7d

Compound **7c** was dissolved in dichloromethane (1 mL), and a solution of hydrogen chloride in 1,4-dioxane (4 M, 0.24 mL) was added. The mixture was reacted at room temperature for 2.5 h. The reaction solution was concentrated to dryness by rotary evaporation, and the residue was dried with an oil pump to obtain the title compound **7d** (45.1 mg, 0.12 mmol), which was directly used in the next step.

MS m/z (ESI): 376.2 [M+1]⁺.

### Step 3

### Tert-butyl ((2S)-2-(4-chlorophenyl)-3-oxo-3-(9-(7-((2-(trimethylsilyl)ethoxy)methyl)-7H-pyrrolo[2,3-d]pyrimidin-4-yl)-3-oxa-7,9-diazabicyclo[3.3.1]nonan-7-yl)propyl)(isopropyl)carbamate 7e

Compound **1e** (47 mg, 0.14 mmol) was dissolved in *N,N*-dimethylformamide (1.2 mL), and N,N-diisopropylethylamine (51.2 mg, 0.40 mmol), compound **7d** (45.1 mg, 0.12 mmol) and *O*-(7-azabenzotriazol-1-yl)-*N*,*N*,*N*',*N*'-tetramethyluronium hexafluorophosphate (68.4 mg, 0.18 mmol) were added. The reaction mixture was stirred at room temperature overnight. The reaction mixture was added with saturated aqueous ammonium chloride solution, extracted with ethyl acetate, washed with saturated sodium chloride solution, dried over anhydrous sodium sulfate, filtered and concentrated to dryness by rotary evaporation, and the residue was dried with an oil pump to obtain the title compound **7e** (83.9 mg, 0.12 mmol), which was used directly in the next step.

MS m/z (ESI): 699.1 [M+1]⁺.

### Step 4

### (2S)-2-(4-chlorophenyl)-1-(9-(7-(hydroxymethyl)-7H-pyrrolo[2,3-d]pyrimidin-4-yl)-3-oxa-7,9-diazabicyclo[3.3.1]nonan-7-yl)-3-(isopropylamino)propan-1-one 7f

Compound **7e** (83.9 mg, 0.12 mmol) was dissolved in dichloromethane (1 mL), and trifluoroacetic acid (1.5 mL) was added. The reaction mixture was stirred at room temperature overnight. The reaction mixture was then concentrated to dryness by rotary evaporation, and the residue was dried with an oil pump to obtain the title compound **7f** (59.9 mg, 0.12 mmol), which was directly used in the next step.

MS m/z (ESI): 499.1 [M+1]⁺.

### Step 5

### (2S)-1-(9-(7H-pyrrolo[2,3-d]pyrimidin-4-yl)-3-oxa-7,9-diazabicyclo[3.3.1]nonan-7-yl)-2-(4-chlorophenyl)-3-(isopropylamino)-propan-1-one 7

Compound **7f** (59.9 mg, 0.12 mmol) was dissolved in water (1 mL) and ethanol (5 mL), and potassium carbonate (166 mg, 1.20 mmol) was added. The reaction mixture was stirred at room temperature for 3 h. The reaction mixture was concentrated by rotary evaporation to remove a part of the solvent, and water and ethyl acetate were added to the residue. The organic phase was separated out, washed with saturated sodium chloride solution, dried over anhydrous sodium sulfate, filtered, and concentrated to dryness by rotary evaporation, and the residue was dissolved in small amount of methanol and then purified by preparative chromatography (instrument model: Gilson 281; chromatographic column: X-Bridge, Prep 30*150 mm; 5 µm, C18; mobile phase: A-water (10 mM ammonium bicarbonate) B-acetonitrile, flow rate: 30 mL/min; column temperature: room temperature) to obtain the title compound 7 (30 mg, yield: 53%).

MS m/z (ESI): 469.1 [M+1]⁺.

¹H NMR (500 MHz, CD₃OD): *δ* 8.09 (d, 1H), 7.17-7.30 (m, 4H), 7.08 (d, 1H), 6.47 (t, 1H), 4.52-4.78 (m, 3H), 4.17-4.26 (m, 1H), 3.87-4.05 (m, 3H), 3.67-3.70 (m, 1H), 3.44 (dd, 1H), 3.16-3.25 (m, 2H), 2.72-2.80 (m, 1H), 2.63-2.71 (m, 2H), 1.22 (d, 6H).

### Example 8

### (S)-2-(4-chlorophenyl)-1-((1R,5S)-3-((5R,7R)-7-fluoro-5-methyl-6,7-dihydro-5H-cyclopenta[d]pyrimidin-4-yl)-3,8-diazabicyclo[3.2.1]octan-8-yl)-3-(isopropylamino)propan-1-one 8

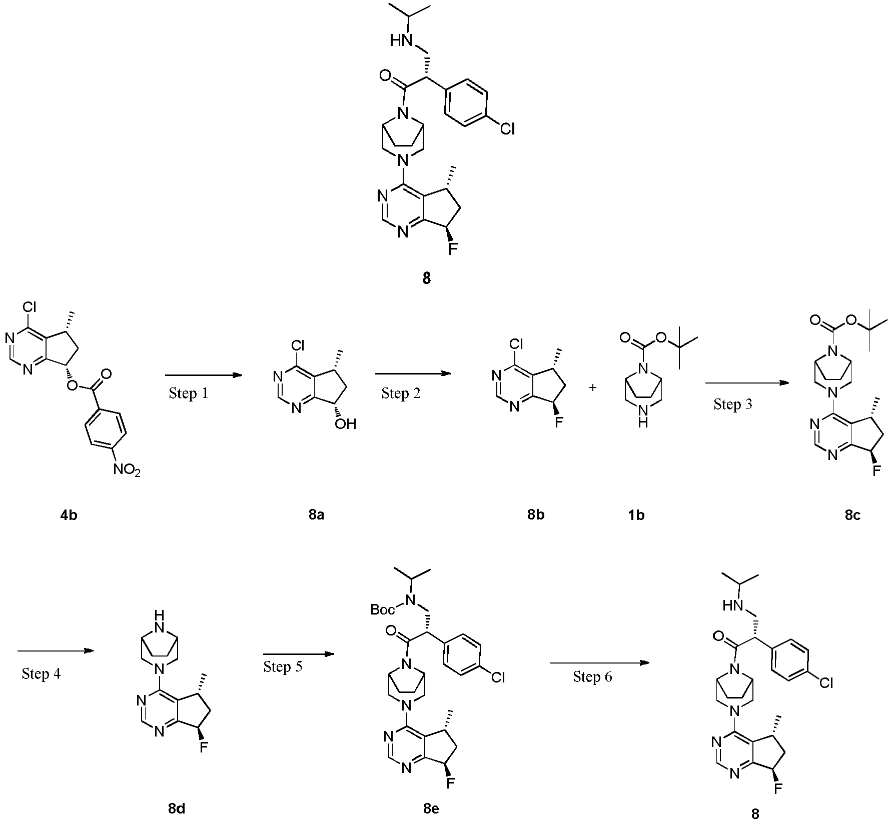

### Step 1

### (5R,7S)-4-chloro-5-methyl-6,7-dihydro-5H-cyclopenta[d]pyrimidin-7-ol 8a

Compound **4b** (1.0 g, 2.996 mmol, prepared according to the method disclosed in page 51 of the specification of patent application "CN104876921A") was dissolved in tetrahydrofuran (10 mL), and lithium hydroxide (180 mg, 7.516 mmol) and water (2 mL) were added at room temperature. The reaction solution was stirred at room temperature for 1 h, then added with water to quench the reaction, extracted with ethyl acetate, dried over anhydrous sodium sulfate and concentrated under reduced pressure. The residue was purified by column chromatography with an eluent system C to obtain the title compound **8a** (430 mg, yield: 77%).

MS m/z (ESI): 185.0 [M+1]⁺.

### Step 2

### (5R,7R)-4-chloro-7-fluoro-5-methyl-6,7-dihydro-5H-cyclopenta[d]pyrimidine 8b

Compound **8a** (430 mg, 2.329 mmol) was dissolved in dry dichloromethane (10 mL), and the mixture was cooled to -20 °C in a dry ice bath. Under N₂ atmosphere, diethylaminosulfur trifluoride (1.12 g, 6.948 mmol) was added dropwise, and after the dropwise addition was completed, the resulting mixture was stirred at -20 °C for 1 h, slowly warmed to room temperature, and then stirred for 2 h. The reaction solution was added with aqueous ammonium chloride solution to quench the reaction, extracted with dichloromethane, dried over anhydrous sodium sulfate, and concentrated under reduced pressure. The residue was purified by column chromatography with an eluent system C to obtain the title compound **8b** (280 mg, yield: 64%).

MS m/z (ESI): 187.0 [M+1]⁺.

### Step 3

### Tert-butyl (1R,5S)-3-((5R,7R)-7-fluoro-5-methyl-6,7-dihydro-5H-cyclopenta[d]pyrimidin-4-yl)-3,8-diazabicyclo[3.2.1]octane-8-carboxylate 8c

Compound **1b** (115 mg, 0.541 mmol, WuXi AppTec Co. Ltd.) was dissolved in n-butanol (5 mL), and **8b** (100 mg, 0.535 mmol) and diisopropylethylamine (208 mg, 1.612 mmol) were added at room temperature. The mixture was reacted at 80 °C for 24 h. The reaction solution was cooled to room temperature and concentrated under reduced pressure, and the residue was purified by column chromatography with an eluent system C to obtain the title compound **8c** (160 mg, yield: 82%). MS m/z (ESI): 364.0 [M+1]⁺.

### Step 4

### (5R,7R)-4-((1R,5S)-3,8-diazabicyclo[3.2.1]octan-3-yl)-7-fluoro-5-methyl-6,7-dihydro-5H-cyclopenta[d]pyrimidine 8d

Compound **8c** (160 mg, 0.441 mmol) was dissolved in a solution of hydrochloric acid in dioxane (4 M, 5 mL), and the mixture was reacted at room temperature for 1 h. The reaction solution was concentrated under reduced pressure to obtain the title compound **8d** (80 mg), which was used directly in the next step without purification.

MS m/z (ESI): 264.0 [M+1]⁺.

### Step 5

### Tert-butyl ((S)-2-(4-chlorophenyl)-3-((1R,5S)-3-((5R,7R)-7-fluoro-5-methyl-6,7-dihydro-5H-cyclopenta[d]pyrimidin-4-yl)-3,8-diazabicyclo[3.2.1]octan-8-yl)-3-oxopropyl)(isopropyl)carbamate 8e

Compound **8d** (80 mg, 0.267 mmol) was dissolved in *N*,*N*'-dimethylformamide (5 mL), and **1e** (92 mg, 0.341 mmol), 2-(7-azabenzotriazol)-tetramethyluronium hexafluorophosphate (102 mg, 0.268 mmol) and diisopropylethylamine (103 mg, 0.798 mmol) were added. The mixture was reacted at room temperature for 2 h. The reaction solution was diluted with ethyl acetate and washed with saturated sodium chloride solution. The organic phase was dried and concentrated under reduced pressure, and the residue was purified by column chromatography with an eluent system C to obtain the title compound **8e** (100 mg, yield: 63%).

MS m/z (ESI): 588.1 [M+1]⁺.

### Step 6

### (S)-2-(4-chlorophenyl)-1-((1R,5S)-3-((5R,7R)-7-fluoro-5-methyl-6,7-dihydro-5H-cyclopenta[d]pyrimidin-4-yl)-3,8-diazabicyclo[3.2. 1]octan-8-yl)-3-(isopropylamino)propan-1-one 8

Compound **8e** (100 mg, 0.17 mmol) was dissolved in a solution of hydrochloric acid in dioxane (4 M, 5 mL), and the mixture was reacted at room temperature for 3 h. The reaction solution was concentrated, and the residue was purified by liquid chromatography (instrument model: Gilson 281; chromatographic column: X-Bridge, Prep 30*150 mm; 5 µm, C18; mobile phase: A-water (10 mM ammonium bicarbonate) B-acetonitrile, flow rate: 30 mL/min; column temperature: room temperature) to obtain the title compound **8** (30 mg, yield: 36%).

MS m/z (ESI): 487.0 [M+1]⁺.

¹H NMR (500 MHz, CD₃OD): δ 8.42-8.48 (m, 1H), 7.34-7.43 (m, 4H), 5.76-5.92 (m, 1H), 4.88-4.94 (m,1H), 4.79-4.83 (m, 1H), 4.62-4.769 (m, 1H), 4.40-4.51 (m, 2H), 3.69-3.71 (m, 1H), 3.49-3.53 (m, 1H), 2.82-2.92 (m, 3H), 2.38-2.42 (m,1H), 2.09-2.25 (m, 2H), 1.52-1.92(m, 3H), 1.25-1.29 (m, 2H), 1.16-1.23 (m, 6H),0.93-0.99 (m, 2H).

### Example 9

### (S)-2-(4-chlorophenyl)-3-(isopropylamino)-1-((1R,5S)-3-(3-methyl-1H-pyrrolo[2,3-b]pyridin-4-yl)-3,8-diazabicyclo[3.2.1]octan-8-yl)propan-1-one 9

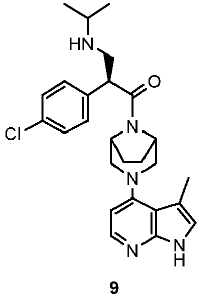

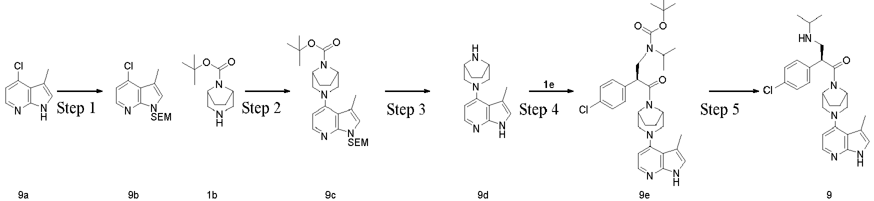

### Step 1

4-chloro-3-methyl-1-((2-(trimethylsilyl)ethoxy)methyl)-1*H*-pyrrolo[2,3-*b*]pyridine **9b** 4-chloro-3-methyl-1*H*-pyrrolo [2,3-b]pyridine **9a** (400 mg, 2.4009 mmol, Bide Pharmatech Ltd.) was dissolved in dry *N*,*N*'-dimethylformamide (8 mL, Adamas), and the mixture was stirred and cooled to 0 °C under nitrogen atmosphere. Sodium hydride (105.6 mg, 2.6403 mmol, 60% purity, Sinopharm) was added, and the resulting mixture was stirred for 10 min. 2-(chloromethoxy)ethyltrimethylsilane (440.3 mg, 2.6409 mmol, 468.4043 uL, Sinopharm) was added, and the resulting mixture was warmed to room temperature and stirred for 1 h. After the completion of the reaction as detected by TLC, the reaction solution was added dropwise slowly into 15 mL of ice water under stirring, and ethyl acetate (10 mL × 3) was added for extraction. The organic phases were combined, dried over anhydrous sodium sulfate, filtered and concentrated, and the residue was purified by column chromatography with a developing solvent system A to obtain the title compound **9b** (550 mg, yield: 77.17%).

MS m/z (ESI): 297.2 [M+1].

### Step 2

### Tert-butyl (1R,5S)-3-(3-methyl-1-((2-(trimethylsilyl)ethoxy)methyl)-1H-pyrrolo[2,3-b]pyridin-4-yl)-3,8-diazabicyclo [3.2.1]octane-8-carboxylate 9c

To a dry microwave tube filled with nitrogen were added compound **9b** (470 mg, 1.5832 mmol), [1,3-bis(2,6-diisopropylbenzene)imidazol-2-ylidene](3-chloropyridine) palladium (II) dichloride (107.9 m, 158.3334 µmol, TCI) and compound **1b** (336.1 mg, 1.5832 mmol), purging with nitrogen was performed to replace the gas in the system, and 1,4-dioxane (4.7 mL, Sinopharm) and lithium bis(trimethylsilyl)amide (3 M, 1.0554 mL, J&K Chemical) were added. The mixture was reacted at 90 °C for 30 min under microwave. The reaction solution was concentrated under reduced pressure, and the residue was purified by column chromatography with a developing solvent system A to obtain the title compound **9c** (374 mg, yield: 49.98%).

MS m/z (ESI): 473.2 [M+1].

### Step 3

### 4-((1R,5S)-3,8-diazabicyclo[3.2.1]octan-3-yl)-3-methyl-1H-pyrrolo[2,3-b]pyridine 9d

Compound **9c** (374 mg, 791.2095 µmol) was dissolved in dichloromethane (6 mL, Sinopharm), and trifluoroacetic acid (1.35 g, 11.87 mmol, Sinopharm) was added. The mixture was stirred at room temperature overnight. The reaction solution was concentrated under reduced pressure to obtain an intermediate compound, and then 5 mL of methanol and 1.5 g of sodium carbonate were added. The resulting mixture was stirred for 3 h and then filtered, and the filtrate was concentrated under reduced pressure to obtain a crude product, which was purified by column chromatography with a developing solvent system A to obtain the title compound **9d** (191 mg, yield: 99.6%).

MS m/z (ESI): 243.1 [M+1].

### Step 4

### Tert-butyl ((S)-2-(4-chlorophenyl)-3-((1R,5S)-3-(3-methyl-1H-pyrrolo[2,3-b]pyridin-4-yl)-3,8-diazabicyclo[3.2.1]octan-8-yl)-3-oxopropyl)(isopropyl)carbamate 9e

Compound **9d** (191 mg, 788 µmol) and compound **1e** (269.4 mg, 788 µmol) were dissolved in 6 mL of dichloromethane, and 2-(7-benzotriazole oxide)-*N*,*N*,*N*',*N*'-tetramethyluronium hexafluorophosphate (389.6 mg, 1.02 mmol, Accela) and triethylamine (239.3 mg, 2.36 mmol, Sinopharm) were added. The mixture was stirred at room temperature overnight. The reaction solution was diluted with ethyl acetate (20 mL), washed with water and concentrated under reduced pressure to obtain the title compound **9e** (crude product, 446 mg), which was used directly in the next step.

MS m/z (ESI): 567.1 [M+1].

### Step 5

### (S)-2-(4-chlorophenyl)-3-(isopropylamino)-1-((1R,5S)-3-(3-methyl-1H-pyrrolo[2,3-b]pyridin-4-yl)-3,8-diazabicyclo[3.2.1]octan-8-yl)propan-1-one 9

Compound **9e** (crude product, 446 mg, 787.8 µmol) was dissolved in ethyl acetate (2 mL), and hydrogen chloride/dioxane solution (6 mL, 4 M, 24 mmol, Energy Chemical) was added. The mixture was stirred at room temperature for 1 h. The reaction solution was concentrated under reduced pressure, and the residue was purified by liquid chromatography (instrument model: Boston; chromatographic column: Phlex, Prep C18 5 µm, 30*150 mm; mobile phase: A-water (10 mmol NH₃HCO₃): B-acetonitrile 35-55% B, flow rate: 30 mL/min; column temperature: room temperature) to obtain the title compound **9** (30 mg, yield: 8.2%).

MS m/z (ESI): 467.1 [M+1].

¹H NMR (500 MHz, CD₃OD): δ 7.89-7.87 (dd, 1H), 7.44-7.40 (m, 3H), 7.30-7.29 (d, 1H), 7.01-6.99 (d, 1H), 6.58-6.44 (dd, 1H), 4.42-4.41 (m, 1H), 4.33-3.94 (m, 5H), 3.88-3.68 (m, 2H), 3.26-3.23 (m, 1H), 2.96-2.94 (m, 1H), 2.82-2.79 (m, 1H), 2.52 (s, 2H), 2.41 (s, 1H), 1.95-1.92 (m, 1H), 1.85-1.76 (m, 1H), 1.57-1.54 (m, 1H), 1.43-1.35 (m, 2H), 1.21-1.12 (m, 6H).

### Test Examples:

### Biological Evaluation

### Test Example 1: Evaluation of Compounds of the Present Disclosure Against AKT1/AKT2/AKT3 in an Enzymatic Assay

The following methods were used to determine the inhibitory effect of the compounds of the present disclosure on the kinase activity of AKT1/AKT2/AKT3 *in vitro.* The experimental methodology is briefly described as follows:
The enzyme activity of AKT1 (Invitrogen, P2999), AKT2 (Invitrogen, PV3184) and AKT3 (Invitrogen, PV3185) was determined using a KinEASE-STK S3 kit (Cisbio, 62ST3PEC). Test compounds were each first subjected to 3-fold gradient dilution with DMSO from 500 µM to obtain a total of 11 concentrations. The 5× buffer in the kit was diluted to 1× buffer, and DTT (Sigma, 43816-10ML) and MgCl₂ were added to make the buffer contain 1 mM DTT and 5 mM MgCl₂. Compounds were each subjected to 20-fold dilution with 1× buffer for later use. The enzyme solution was obtained by diluting the AKT1/AKT2/AKT3 kinase with 1× buffer. ATP (Invitrogen, PV3227) and S3-biotin in the kit were diluted with 1× buffer to obtain a substrate-ATP mixture solution for later use. 2 µL of the enzyme solution and 4 µL of the compound solution were added to each well of a 384-well plate (Corning, 4513), and the mixture was incubated at room temperature for 30 min, followed by addition of 4 µL of the ATP and S3-biotin mixture solution. The resulting mixture was incubated at room temperature for 90 min. AKT1 enzyme reaction conditions were a final concentration of 2 nM for enzyme, a final concentration of 10 µM for ATP, and a final concentration of 2 µM for S3-biotin. AKT2 enzyme reaction conditions were a final concentration of 5 nM for enzyme, a final concentration of 10 µM for ATP, and a final concentration of 2 µM for S3-biotin. AKT3 enzyme reaction conditions were a final concentration of 0.4 nM for enzyme, a final concentration of 45 µM for ATP, and a final concentration of 2 µM for S3-biotin. The detection solution was prepared by diluting the S3-cryptate and streptavidin-XL665 with the detection buffer in the kit. After incubation, 10 µL of the detection solution was added to each well, the final concentration of S3-cryptate was a concentration obtained by 200-fold dilution of the stock solution, and the final concentration of streptavidin-XL665 was 125 nM. The mixture was incubated at room temperature for 60 min, values of signals emitted at 650 nm and 620 nm after excitation at 337 nm were measured by using an HTRF module of a multi-functional microplate detector (BMG Labtech, PHERAstar FS), and the reading ratio was multiplied by 10,000 to obtain a specific value. Dose-response curves were drawn according to the concentration of the compounds and the specific values by using Graphpad Prism software, and the IC₅₀ values for the inhibitory activity of the compounds were calculated.

### Experimental data

The inhibitory activity of the compounds of the present disclosure against AKT1/AKT2/AKT3 enzymes was determined by the above assay, and the IC₅₀ values measured are shown in Table 1.

**Table 1. IC₅₀ values for AKT1/AKT2/AKT3 enzyme inhibition by compounds of the present disclosure**

| Example No. | AKT1 IC₅₀/nM | AKT2 IC₅₀/nM | AKT3 IC₅₀/nM |
|---|---|---|---|
| 1 | 20. | 64.9 | 27.6 |
| 2 | 60 | 74.1 | 2.0 |
| 3 | 5.2 | 3.9 | 1.2 |
| 4 | 38.6 | 46.4 | 55.6 |
| 5 | 68.1 | 70.1 | 112 |
| 6 | 88.4 | 140.9 | 268.3 |
| 7 | 82 | 270 | 60 |
| 8 | 56.2 | 54 | 66 |
| 9 | 19.6 | 13.1 | 6.3 |

Conclusion: the compounds of the present disclosure all have a good inhibitory effect on AKT1/AKT2/AKT3 enzymes.

### Pharmacokinetic Study of Compounds of Present Disclosure

### Pharmacokinetic Study of Compounds of Present Disclosure in Nude Mice

### 1. Abstract

The drug concentration in the plasma of the test animals (Balb/c nude mice) at different time points after intragastric administration (i.g.) of the test compounds was determined by using an LC/MS/MS method. The pharmacokinetic performance of the compounds of the present disclosure in nude mice was studied and their pharmacokinetic profile was evaluated.

### 2. Methodology

### 2.1. Test compounds

Example 2 and Example 4.

### 2.2. Test animals

18 Balb/c nude mice, female, divided into 2 groups and purchased from Vital River Laboratory Animal Technology Co., Ltd.

### 2.3. Drug preparation

A certain amount of compound was weighed out, 99.8% 0.5% methylcellulose was added into the compound, and then 0.2% Tween was added. The mixture was subjected to ultrasonic treatment to obtain a suspension, which was stirred for administration.

### 2.4. Administration

The nude mice were subjected to intragastric administration, the administration dose was 50 mg/kg or 100 mg/kg, and the administration volume was 0.2 mL/10 g.

### 3. Procedures

The test compound was administered intragastrically to the nude mice, and 0.1 mL of blood was collected before administration and 0.25 h, 0.5 h, 1.0 h, 2.0 h, 4.0 h, 6.0 h, 8.0 h, 11.0 h and 24.0 h after administration. The blood was placed in an EDTA-K2 anticoagulation tube and centrifuged at 10,000 rpm for 1 min (4 °C), and plasma was separated out within 1 h and then stored at -20 °C for testing. The process from blood sampling to centrifugation was performed under an ice bath.

The content of the test compounds at different concentrations in the nude mice plasma after intragastric administration was determined: 20 µL of nude mouse plasma at each time point after administration was mixed with 50 µL of internal standard solution (camptothecin, 100 ng/mL) and 200 µL of acetonitrile, and the mixture was vortexed for 5 min and centrifuged for 10 min at 3700 rpm. 1 µL of supernatant of the plasma sample was taken for LC/MS/MS analysis.

### 4. Pharmacokinetic parameters

**Table 2. Pharmacokinetic parameters of compounds of the present disclosure**

| No. | Dose (mg/kg) | Plasma concentration | Area under curve | Half-life | Residence time | Clearance | Apparent volume of distribution |
|---|---|---|---|---|---|---|---|
| | | Cmax (ng /mL) | AUC₀₋ₜ (ng /mL*h) | T_{1/2} (h) | MRT(h) | CUF (ml/min/kg) | Vz/F (ml/kg) |
| Example 2 | 50 | 3000 | 20207 | 2.3 | 4.9 | 41.2 | 8029 |
| | 100 | 3500 | 35990 | 4.1 | 6.7 | 45.4 | 16213 |
| Example 4 | 50 | 4133 | 14307 | 1.8 | 3.0 | 57.5 | 8705 |
| | 100 | 5497 | 27527 | 2.5 | 3.7 | 60.5 | 13180 |

Conclusion: the compounds of the present disclosure demonstrate are well absorbed, and as the dose increases, the absorption increases accordingly.

### Pharmacokinetic Study of Compounds of Present Disclosure in Rats

### 1. Abstract

The drug concentration in the plasma of the test animals (SD rats) at different time points after injection (i.v.) of the test compounds was determined by using an LC/MS/MS method. The pharmacokinetic performance in rats of the compounds of present disclosure was studied and their pharmacokinetic profile was evaluated.

### 2. Methodology

### 2.1. Test compounds

Example 2 and Example 4.

### 2.2. Test animals

8 SD rats, half male and half female, divided into 2 groups, purchased from Vital River Laboratory Animal Technology Co., Ltd.

### 2.3. Drug preparation

A certain amount of the compound was weighed out, and 5% of DMSO, 5% of Tween 80 and 90% of normal saline were added to obtain a colorless and clear solution.

### 2.4. Administration

The rats were subjected to administration by injection, the administration dose was 1 mg/kg, and the administration volume was 5 mL/kg.

### 3. Procedures

The test compound was administered by injection to the rats, and 0.2 mL of blood was collected from the orbit before administration and 5 min, 0.25 h, 0.5 h, 1.0 h, 2.0 h, 4.0 h, 8.0 h, 11.0 h and 24.0 h after administration. The blood was placed in an EDTA-K2 anticoagulation tube and centrifuged at 10,000 rpm for 1 min (4 °C), and plasma was separated out within 1 h and then stored at -20 °C for testing. The process from blood sampling to centrifugation was performed under an ice bath.

The content of the test compounds at different concentrations in the rat plasma after administration by injection was determined: 25 µL of rat plasma at each time point after administration was mixed with 50 µL of internal standard solution (camptothecin, 100 ng/mL) and 200 µL of acetonitrile, and the mixture was vortexed for 5 min and centrifuged for 10 min at 3700 rpm. 3 µL of supernatant of the plasma sample was taken for LC/MS/MS analysis.

### 4. Pharmacokinetic parameters

**Table 3. Pharmacokinetic parameters of compounds of the present disclosure**

| No. | Dose (mg/kg) | Area under curve | Half-life | Residence time | Clearance | Apparent volume of distribution |
|---|---|---|---|---|---|---|
| | | AUC₀₋ₜ (ng /mL^{∗}h) | T_{1/2} (h) | MRT(h) | CL (ml/min/kg) | Vz(ml/kg) |
| Example 2 | 1 | 221 | 2.0 | 2.6 | 75.4 | 12962 |
| Example 4 | 1 | 205 | 2.5 | 2.2 | 82.9 | 16341 |

Conclusion: the compounds of the present disclosure have good pharmacokinetic performance.

## Claims

1. A compound of general formula (I) or a tautomer, mesomer, racemate, enantiomer or diastereomer thereof or a mixture thereof, or a pharmaceutically acceptable salt thereof: 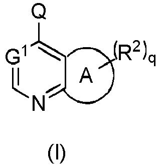 wherein:
Q is a group of formula (Qa) or (Qb): 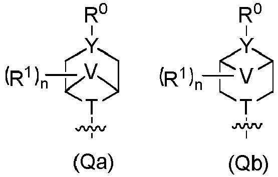
V is selected from the group consisting of -CH₂-, -C(CH₃)₂-, -CH₂CH₂-, -CH₂CH₂CH₂-, -CH₂OCH₂-, -CH₂SCH₂-, -CH₂S(O)CH₂-, -CH₂S(O)₂CH₂- and -CH₂N(R^{a})CH₂-;
Y is an N atom or CR³;
T is CH or an N atom; provided that when Y is CR³, T is an N atom;
R⁰ is -C(O)CHR⁵R⁶ or -C(O)NHCHR⁵R⁶;
R¹ are identical or different and are each independently selected from the group consisting of a hydrogen atom, halogen, alkyl, hydroxyalkyl, alkoxy, haloalkoxy and haloalkyl;
ring A is 5-membered heterocyclyl, 5-membered cycloalkyl or 5-membered heteroaryl;
G¹ is selected from the group consisting of CR⁴ and an N atom;
R² are identical or different and are each independently selected from the group consisting of a hydrogen atom, oxo, halogen, alkyl, alkenyl, alkynyl, alkoxy, haloalkyl, haloalkoxy, cyano, amino, nitro, hydroxy, hydroxyalkyl, cycloalkyl, heterocyclyl, aryl and heteroaryl;
R³ is selected from the group consisting of a hydrogen atom, halogen, alkyl, alkenyl, alkynyl, alkoxy, haloalkyl, haloalkoxy, cyano, amino, -NR⁷R⁸, nitro, hydroxy, hydroxyalkyl, cycloalkyl, heterocyclyl, aryl and heteroaryl, wherein the alkyl is optionally substituted with one or more substituents selected from the group consisting of amino, -NR⁷R⁸, halogen, alkoxy, haloalkyl, cyano, nitro, hydroxy, hydroxyalkyl, cycloalkyl, heterocyclyl, aryl and heteroaryl;
R^{a} is selected from the group consisting of a hydrogen atom, alkyl, haloalkyl, hydroxyalkyl, cycloalkyl, heterocyclyl, aryl and heteroaryl;
R⁴ is selected from the group consisting of a hydrogen atom, halogen, alkyl, alkenyl, alkynyl, alkoxy, haloalkyl, haloalkoxy, cyano, amino, nitro, hydroxy, hydroxyalkyl, cycloalkyl, heterocyclyl, aryl and heteroaryl;
R⁵ is selected from the group consisting of halogen, alkyl, alkenyl, alkynyl, alkoxy, haloalkyl, haloalkoxy, cyano, amino, nitro, hydroxy, hydroxyalkyl, cycloalkyl, heterocyclyl, aryl and heteroaryl, wherein the alkyl, alkenyl, alkynyl, alkoxy, hydroxyalkyl, cycloalkyl, heterocyclyl, aryl and heteroaryl are each independently optionally substituted with one or more substituents selected from the group consisting of -NR⁹R¹⁰, oxo, halogen, alkyl, alkenyl, alkynyl, alkoxy, haloalkyl, cyano, amino, nitro, hydroxy, hydroxyalkyl, cycloalkyl, heterocyclyl, aryl and heteroaryl;
R⁶ is selected from the group consisting of halogen, alkyl, alkenyl, alkynyl, alkoxy, haloalkyl, haloalkoxy, cyano, amino, nitro, hydroxy, hydroxyalkyl, cycloalkyl, heterocyclyl, aryl and heteroaryl, wherein the alkyl, alkenyl, alkynyl, alkoxy, hydroxyalkyl, cycloalkyl, heterocyclyl, aryl and heteroaryl are each independently optionally substituted with one or more substituents selected from the group consisting of oxo, halogen, alkyl, alkenyl, alkynyl, alkoxy, haloalkyl, cyano, amino, nitro, hydroxy, hydroxyalkyl, cycloalkyl, heterocyclyl, aryl and heteroaryl;
R⁷ and R⁸ are identical or different and are each independently selected from the group consisting of a hydrogen atom, halogen, alkyl, alkenyl, alkynyl, alkoxy, haloalkyl, haloalkoxy, cyano, amino, nitro, hydroxy, hydroxyalkyl, cycloalkyl, heterocyclyl, aryl and heteroaryl;
R⁹ and R¹⁰ are identical or different and are each independently selected from the group consisting of a hydrogen atom, halogen, alkyl, alkenyl, alkynyl, alkoxy, haloalkyl, haloalkoxy, cyano, amino, nitro, hydroxy, hydroxyalkyl, cycloalkyl, cycloalkylalkyl, heterocyclyl, aryl and heteroaryl;
n is 0, 1, 2, 3 or 4; and
q is 0, 1, 2, 3, 4 or 5.

2. The compound of general formula (I) or the tautomer, mesomer, racemate, enantiomer or diastereomer thereof or the mixture thereof, or the pharmaceutically acceptable salt thereof according to claim 1, being a compound of general formula (IIaa), (IIbb) or (IIcc) or a tautomer, mesomer, racemate, enantiomer or diastereomer thereof or a mixture thereof, or a pharmaceutically acceptable salt thereof, 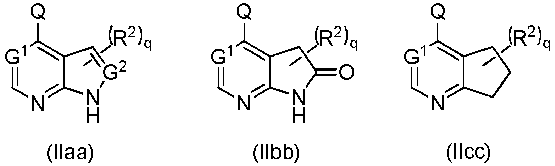 wherein:
G² is CR⁴ or an N atom; and
Q, G¹, R², R⁴ and q are defined as in claim 1.

3. The compound of general formula (I) or the tautomer, mesomer, racemate, enantiomer or diastereomer thereof or the mixture thereof, or the pharmaceutically acceptable salt thereof according to claim 1 or 2, being a compound of general formula (IIcc-1) or a tautomer, mesomer, racemate, enantiomer or diastereomer thereof or a mixture thereof, or a pharmaceutically acceptable salt thereof: 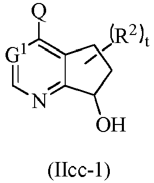 wherein:
t is 0, 1, 2, 3 or 4; and
Q, G¹ and R² are defined as in claim 1.

4. The compound of general formula (I) or the tautomer, mesomer, racemate, enantiomer or diastereomer thereof or the mixture thereof, or the pharmaceutically acceptable salt thereof according to any one of claims 1 to 3, wherein Q is the group of formula (Qb):

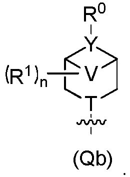

5. The compound of general formula (I) or the tautomer, mesomer, racemate, enantiomer or diastereomer thereof or the mixture thereof, or the pharmaceutically acceptable salt thereof according to any one of claims 1 to 4, wherein V is selected from the group consisting of -CH₂-, -C(CH₃)₂-, -CH₂CH₂-, -CH₂CH₂CH₂- and -CH₂OCH₂-, preferably selected from the group consisting of -CH₂-, -C(CH₃)₂-, -CH₂CH₂- and -CH₂CH₂CH₂-.

6. The compound of general formula (I) or the tautomer, mesomer, racemate, enantiomer or diastereomer thereof or the mixture thereof, or the pharmaceutically acceptable salt thereof according to any one of claims 1 to 5, wherein T is an N atom.

7. The compound of general formula (I) or the tautomer, mesomer, racemate, enantiomer or diastereomer thereof or the mixture thereof, or the pharmaceutically acceptable salt thereof according to any one of claims 1 to 6, wherein Y is an N atom, T is an N atom, and V is selected from the group consisting of -CH₂CH₂-, -CH₂CH₂CH₂- and -CH₂OCH₂-; preferably, Y is an N atom, T is an N atom, and V is -CH₂CH₂-.

8. The compound of general formula (I) or the tautomer, mesomer, racemate, enantiomer or diastereomer thereof or the mixture thereof, or the pharmaceutically acceptable salt thereof according to any one of claims 1 to 7, wherein G¹ is CH or an N atom, preferably an N atom.

9. The compound of general formula (I) or the tautomer, mesomer, racemate, enantiomer or diastereomer thereof or the mixture thereof, or the pharmaceutically acceptable salt thereof according to any one of claims 2 and 4 to 8, wherein G² is CR⁴; R⁴ is defined as in claim 2.

10. The compound of general formula (I) or the tautomer, mesomer, racemate, enantiomer or diastereomer thereof or the mixture thereof, or the pharmaceutically acceptable salt thereof according to any one of claims 1 to 9, wherein R⁴ is a hydrogen atom.

11. The compound of general formula (I) or the tautomer, mesomer, racemate, enantiomer or diastereomer thereof or the mixture thereof, or the pharmaceutically acceptable salt thereof according to any one of claims 1, 2 and 4 to 9, being a compound of general formula (IIIaa), (IIIbb) or (IIIcc) or a tautomer, mesomer, racemate, enantiomer or diastereomer thereof or a mixture thereof, or a pharmaceutically acceptable salt thereof: 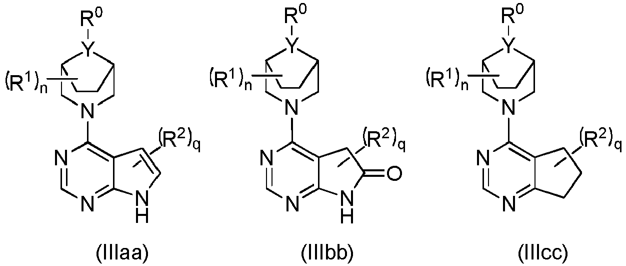 wherein:
Y, R°, R¹, R², q and n are defined as in claim 1.

12. The compound of general formula (I) or the tautomer, mesomer, racemate, enantiomer or diastereomer thereof or the mixture thereof, or the pharmaceutically acceptable salt thereof according to any one of claims 1 to 11, wherein R⁰ is -C(O)CHR⁵R⁶, wherein R⁵ and R⁶ are defined as in claim 1; preferably, R⁰ is -C(O)CHR⁵R⁶, wherein R⁵ is alkyl, wherein the alkyl is optionally substituted with one or more substituents selected from the group consisting of hydroxy and -NR⁹R¹⁰, R⁹ and R¹⁰ are identical or different and are each independently a hydrogen atom or alkyl; R⁶ is aryl or heteroaryl, wherein the aryl or heteroaryl is optionally substituted with one or more substituents selected from the group consisting of halogen, alkyl, alkoxy, haloalkyl and haloalkoxy.

13. The compound of general formula (I) or the tautomer, mesomer, racemate, enantiomer or diastereomer thereof or the mixture thereof, or the pharmaceutically acceptable salt thereof according to claim 1, being a compound of general formula (IV) or a tautomer, mesomer, racemate, enantiomer or diastereomer thereof or a mixture thereof, or a pharmaceutically acceptable salt thereof: 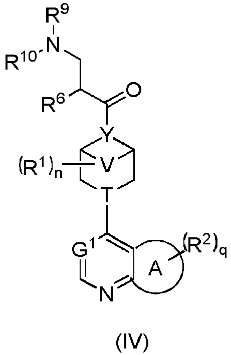 wherein:
R¹, R², R⁶, R⁹, R¹⁰, G¹, ring A, Y, V, T, q and n are defined as in claim 1.

14. The compound of general formula (I) or the tautomer, mesomer, racemate, enantiomer or diastereomer thereof or the mixture thereof, or the pharmaceutically acceptable salt thereof according to any one of claims 1 to 13, wherein Y is an N atom.

15. The compound of general formula (I) or the tautomer, mesomer, racemate, enantiomer or diastereomer thereof or the mixture thereof, or the pharmaceutically acceptable salt thereof according to any one of claims 1 to 14, wherein R¹ is a hydrogen atom.

16. The compound of general formula (I) or the tautomer, mesomer, racemate, enantiomer or diastereomer thereof or the mixture thereof, or the pharmaceutically acceptable salt thereof according to any one of claims 1 to 15, wherein R² are identical or different and are each independently selected from the group consisting of a hydrogen atom, oxo, halogen, alkyl, haloalkyl, hydroxyalkyl, hydroxy, alkoxy and haloalkoxy;
preferably, R² are identical or different and are each independently selected from the group consisting of a hydrogen atom, oxo, halogen, C₁₋₆ alkyl, C₁₋₆ haloalkyl, C₁₋₆ hydroxyalkyl, hydroxy, C₁₋₆ alkoxy and C₁₋₆ haloalkoxy.

17. The compound of general formula (I) or the tautomer, mesomer, racemate, enantiomer or diastereomer thereof or the mixture thereof, or the pharmaceutically acceptable salt thereof according to any one of claims 1 to 16, selected from the group consisting of any one of the following compounds:

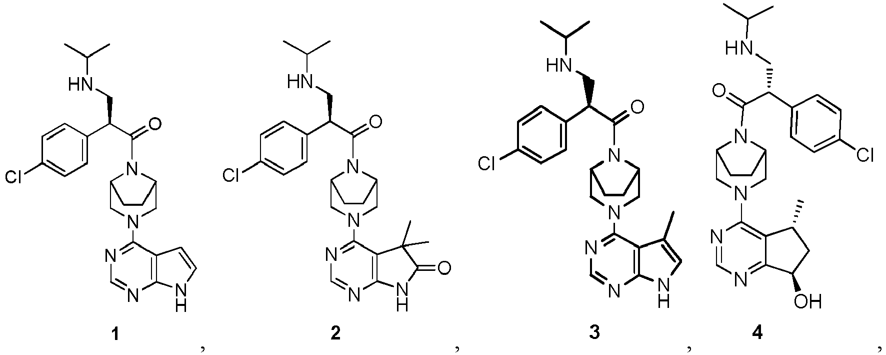

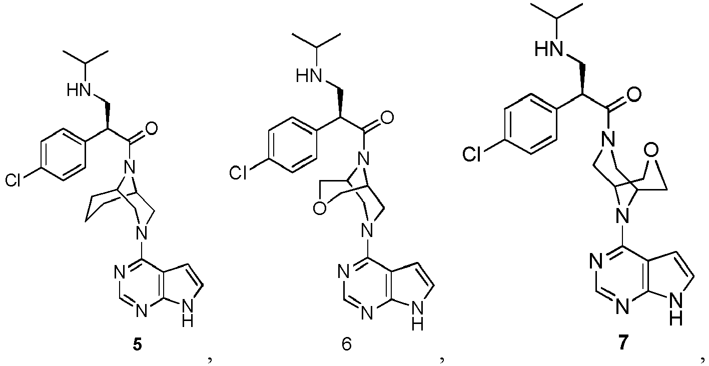

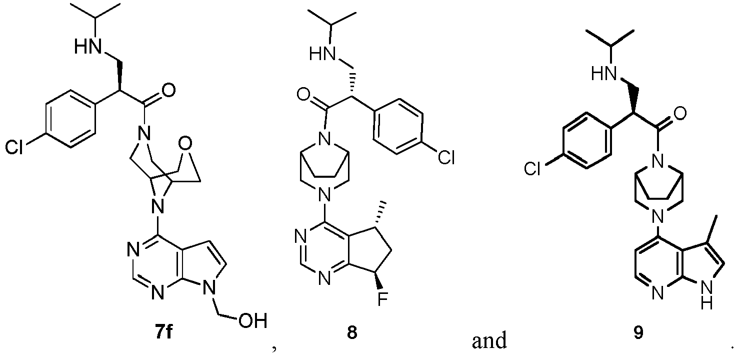

18. A compound of general formula (IVA) or a tautomer, mesomer, racemate, enantiomer or diastereomer thereof or a mixture thereof, or a pharmaceutically acceptable salt thereof, 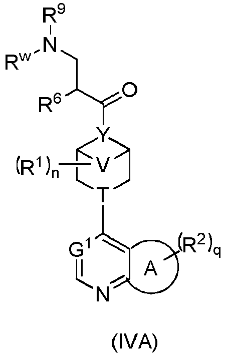 wherein:
R^{w} is an amino protecting group, preferably tert-butoxycarbonyl;
R¹, R², R⁶, R⁹, G¹, ring A, Y, V, T, n and q are defined as in claim 13.

19. The compound or the tautomer, mesomer, racemate, enantiomer or diastereomer thereof or the mixture thereof, or the pharmaceutically acceptable salt thereof according to claim 18, selected from the group consisting of any one of the following compounds:

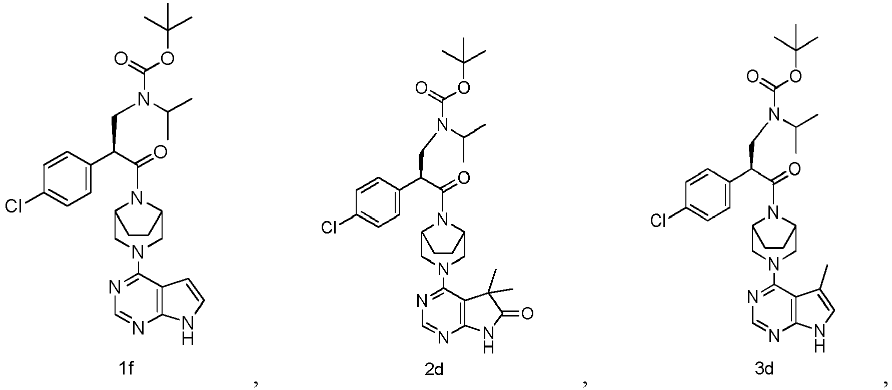

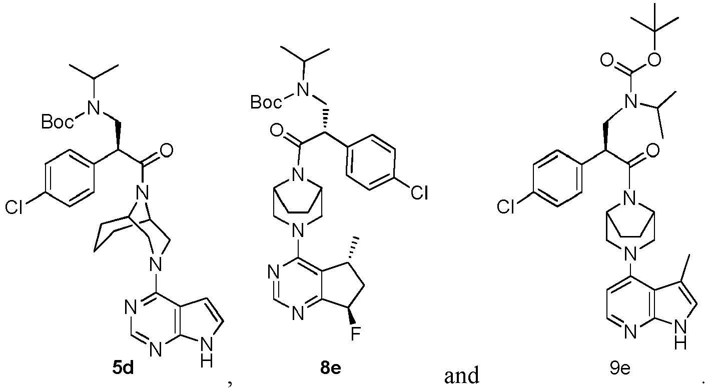

20. A compound of general formula (IIcc-1A) or a tautomer, mesomer, racemate, enantiomer or diastereomer thereof or a mixture thereof, or a pharmaceutically acceptable salt thereof, 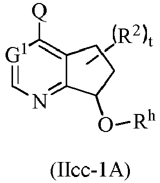 wherein:
R^{h} is a hydroxy protecting group, preferably *p*-nitrobenzoyl;
t is 0, 1, 2, 3 or 4; and
G¹, Q and R² are defined as in claim 3.

21. The compound or the tautomer, mesomer, racemate, enantiomer or diastereomer thereof or the mixture thereof, or the pharmaceutically acceptable salt thereof according to claim 20, being the following compound:

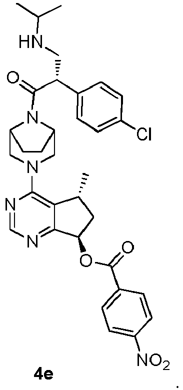

22. A method for preparing the compound of general formula (IV) or the tautomer, mesomer, racemate, enantiomer or diastereomer thereof or the mixture thereof, or the pharmaceutically acceptable salt thereof according to claim 13, comprising: 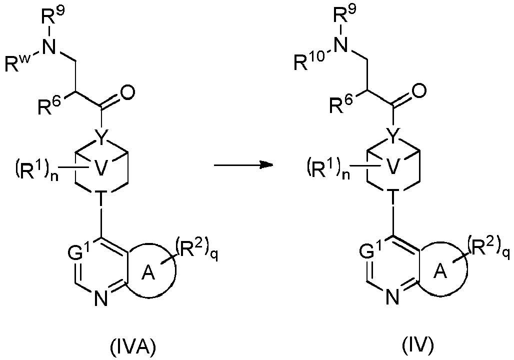
removing an amino protecting group from a compound of general formula (IVA) to obtain the compound of general formula (IV),
wherein:
R^{w} is the amino protecting group, preferably *tert*-butoxycarbonyl;
R¹⁰ is a hydrogen atom; and
R¹, R², R⁶, R⁹, Y, V, T, ring A, G¹, n and q are defined as in claim 13.

23. A method for preparing the compound of general formula (IIcc-1) or the tautomer, mesomer, racemate, enantiomer or diastereomer thereof or the mixture thereof, or the pharmaceutically acceptable salt thereof according to claim 3, comprising: 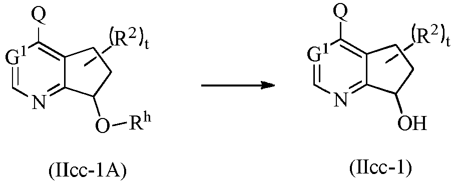
removing a hydroxy protecting group R^{h} from a compound of general formula (IIcc-1A) to obtain the compound of general formula (IIcc-1),
wherein:
R^{h} is the hydroxy protecting group, preferably *p*-nitrobenzoyl; and
G¹, Q, R² and t are defined as in claim 3.

24. A pharmaceutical composition, comprising the compound of general formula (I) or the tautomer, mesomer, racemate, enantiomer or diastereomer thereof or the mixture thereof, or the pharmaceutically acceptable salt thereof according to any one of claims 1 to 17, and one or more pharmaceutically acceptable carriers, diluents or excipients.

25. Use of the compound of general formula (I) or the tautomer, mesomer, racemate, enantiomer or diastereomer thereof or the mixture thereof, or the pharmaceutically acceptable salt thereof according to any one of claims 1 to 17, or the pharmaceutical composition according to claim 24 in preparing a medicament for inhibiting AKT1/2/3 (AKT pan).

26. Use of the compound of general formula (I) or the tautomer, mesomer, racemate, enantiomer or diastereomer thereof or the mixture thereof, or the pharmaceutically acceptable salt thereof according to any one of claims 1 to 17, or the pharmaceutical composition according to claim 24 in preparing a medicament for treating and/or preventing a tumor, wherein the tumor is preferably cancer.

27. Use of the compound of general formula (I) or the tautomer, mesomer, racemate, enantiomer or diastereomer thereof or the mixture thereof, or the pharmaceutically acceptable salt thereof according to any one of claims 1 to 17, or the pharmaceutical composition according to claim 24 in preparing a medicament for treating or preventing a disease or disorder, wherein the disease or disorder is selected from the group consisting of ovarian cancer, breast cancer, prostate cancer, neuroglioma, glioblastoma, gastric cancer, fallopian tube cancer, lung cancer, peritoneal tumor, melanoma, brain cancer, esophageal cancer, liver cancer, pancreatic cancer, colorectal cancer, lung cancer, kidney cancer, cervical cancer, skin cancer, neuroblastoma, sarcoma, bone cancer, uterine cancer, endometrial cancer, head and neck tumor, multiple myeloma, lymphoma, non-Hodgkin's lymphoma, non-small cell lung cancer, polycythemia vera, leukemia, thyroid tumor, bladder cancer and gallbladder cancer.
